(19) Europäisches Patentamt
European Patent Office
Office européen des brevets

(11) Numéro de publication: **0 295 474 B1**

(12) **FASCICULE DE BREVET EUROPEEN**

(45) Date de publication de fascicule du brevet: **17.07.91**

(51) Int. Cl.⁵: **C07C 215/20**, A61K 7/13

(21) Numéro de dépôt: **88108493.3**

(22) Date de dépôt: **27.05.88**

# (54) Nouvelles chlorométaphénylènediamines, leur utilisation en tant que coupleurs en teinture d'oxydation des fibres kératiniques, compositions tinctoriales pour cheveux contenant ces composés et procédé de teinture utilisant lesdites compositions.

(30) Priorité: **29.05.87 LU 86904**

(43) Date de publication de la demande: **21.12.88 Bulletin 88/51**

(45) Mention de la délivrance du brevet: **17.07.91 Bulletin 91/29**

(84) Etats contractants désignés: **AT BE CH DE ES FR GB GR IT LI NL SE**

(56) Documents cités:
**EP-A- 0 075 242**

**CHEMICAL ABSTRACTS, vol. 64, no. 13, 20 juin 1966, colonne 19553a-c, Columbus, Ohio, US; T. KAMETANI et al.: "Syntheses of heterocyclic compounds. CXXIX. Streptonigrin and related compounds. 2. Syntheses of 7-aminoquinoline derivatives from hexachlorocyclohexane"**

(73) Titulaire: **L'OREAL**
**14, Rue Royale**
**F-75008 Paris(FR)**

(72) Inventeur: **Junino, Alex**
**16 Rue du Docteur Bergonié**
**F-93180 Livry-Gargan(FR)**
Inventeur: **Vandenbossche, Jean-Jacques**
**6 Rue Léon Richet**
**F-93600 Aulnay-sous-Bois(FR)**
Inventeur: **Borowiak, Hervé**
**84, 8ème avenue**
**F-93290 Tremblay-les-Gonesse(FR)**
Inventeur: **Lang, Gérard**
**44 Avenue Lacour**
**F-95210 Saint-Gratien(FR)**

(74) Mandataire: **Casalonga, Axel**
**BUREAU D.A. CASALONGA - JOSSE Morassistrasse 8**
**W-8000 München 5(DE)**

**EP 0 295 474 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouvelles chlorométaphénylènediamines, leur utilisation en tant que coupleurs en teinture d'oxydation des fibres kératiniques, les compositions tinctoriales pour cheveux humains contenant ces composés à titre de coupleurs en association avec des précurseurs de colorants d'oxydation et un procédé de teinture utilisant lesdites compositions.

Dans le domaine des fibres kératiniques telles que les cheveux ou les fourrures, les métaphénylènediamines jouent un rôle important connu depuis longtemps. Elles font partie de la classe des composés couramment appelés coupleurs.

Ces coupleurs sont utilisés pour faire varier les nuances obtenues avec les précurseurs de colorants d'oxydation, notamment de type para, tels que les paraphénylènediamines et les para-aminophénols.

Généralement, l'association des métaphénylènediamines avec les paraphénylènediamines en milieu alcalin oxydant, et plus particulièrement en présence d'eau oxygénée, donne naissance à des composés capables de conférer aux fibres kératiniques des colorations bleues très puisssantes.

L'association des métaphénylènediamines avec les paraaminophénols en milieu alcalin oxydant, et plus particulièrement en présence d'eau oxygénée, donne habituellement naissance à des composés capables de conférer aux fibres kératiniques des colorations rouges.

Or, la demanderesse a constaté que, contrairement à toute attente, certaines métaphénylènediamines, lorsqu'elles sont associées avec des paraphénylènediamines en milieu alcalin et plus particulièrement en présence d'eau oxygénée, permettent d'obtenir sur les fibres kératiniques des nuances inattendues, pouvant être des nuances beige à brun clair ou pourpre plus ou fins rouge. Par couplage avec les para-aminophénols, ces métaphénylènediamines conduisent à des couleurs brun clair et jaune non dénuées d'intérêt pour l'homme de l'art dans le cas où l'on souhaite obtenir des nuances claires ou rabattre des rouges trop vifs.

La présente invention a donc pour objet les composés nouveaux répondant à la formule (I) ci-dessous ou leurs sels d' addition avec un acide

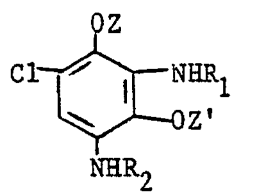

(I)

formule dans laquelle :

Z et Z' représentent indépendamment l'un de l'autre un radical alkyle ayant de 1 à 4 atomes de carbone, ou hydroxyalkyle ayant de 2 à 4 atomes de carbone,

$R_1$ et $R_2$ représentent indépendamment l'un de l'autre un atome d'hydrogène, un radical alkyle ayant de 1 à 4 atomes de carbone ou mono- ou polyhydroxyalkyle ayant de 2 à 3 atomes de carbone, à condition que lorsque $R_1$ et $R_2$ désignent simultanément un atome d'hydrogène, Z et Z' ne désignent pas simultanément le radical méthyle.

Un autre objet de l'invention est l'utilisation à titre de coupleurs des composés de formule (I')

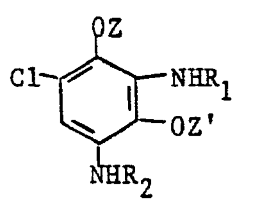

(I')

dans laquelle :

Z et Z' représentent indépendamment l'un de l'autre un radical alkyle ayant de 1 à 4 atomes de carbone ou hydroxyalkyle ayant de 2 à 4 atomes de carbone, $R_1$ et $R_2$ représentent indépendamment l'un de l'autre un atome d'hydrogène, un radical alkyle ayant de 1 à 4 atomes de carbone ou mono- ou polyhydroxyalkyle

ayant de 2 à 3 atomes de carbone, ou de leurs sels d'addition avec un acide, en association avec des précurseurs de colorants d'oxydation, pour la teinture des fibres kératiniques et en particulier des cheveux humains.

La présente invention a également pour objet une composition tinctoriale pour fibres kératiniques et en particulier pour cheveux humains comprenant, dans un support aqueux cosmétiquement acceptable, au moins un composé de formule (I′) ci-dessus ou bien l'un de ses sels d'acides, à titre de coupleur, en association avec au moins un précurseur de colorant d'oxydation de type para.

L'invention vise aussi un procédé de teinture capillaire utilisant une telle composition tinctoriale.

Selon la signification des radicaux $R_1$ et $R_2$, les composés de formule (I) ou (I′) peuvent être préparés selon les procédés décrits ci-dessous.

1°) Procédé de préparation des composés (I) ou (I′) dans lesquels $R_1 = R_2 = H$

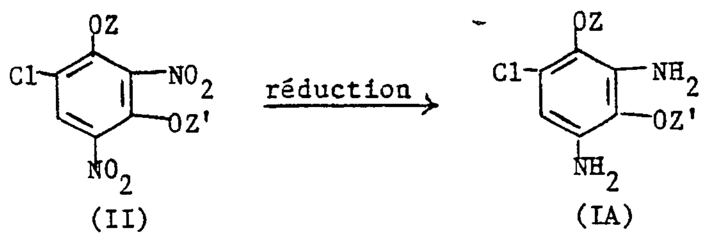


Z et Z′ ont les significations indiquées ci-dessus pour la formule (I′).

Les composés de formule (II) sont réduits par le fer en milieu acide acétique à une température comprise entre 50° C et 100° C.

2°) Procédé de préparation des composés (I) ou (I′) dans lesquels $R_1 = R_2 \neq H$ et ont les significations indiquées dans la formule (I).

On soumet les composés ($I_A$) ci-dessus à une réaction d'alkylation ou d'hydroxyalkylation selon des procédés classiques d'alkylation ou d'hydroxyalkylation des amines aromatiques.

3°) Procédé de préparation des composés (I) ou (I′) dans lesquels $R_1 = H$ et $R_2 \neq H$ (composés IB)

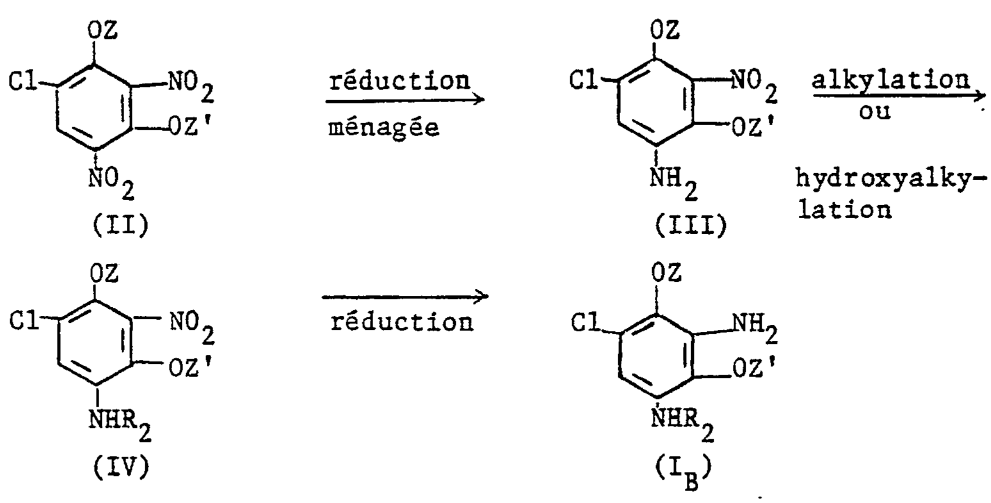


Z, Z′ et $R_2$ ont les significations indiquées dans la formule (I).

Le composé de formule (II) est soumis à une réduction ménagée. Parmi les réductions ménagées connues, on peut citer une réduction par transfert d'hydrogène en présence d'un catalyseur tel que le

palladium sur charbon et de cyclohexène comme donneur d'hydrogène.

Le composé de formule (III) ainsi obtenu est soumis à une réaction d'alkylation ou d'hydroxyalkylation et conduit au composé (IV).

Le composé (IV) est réduit par le fer en présence d'acide acétique à une température comprise entre 50° C et 100° C.

4°) Procédé de préparation des composés (I) ou (I′) dans lesquels $R_1 \neq H$ et $R_2 = H$ (composés IC)

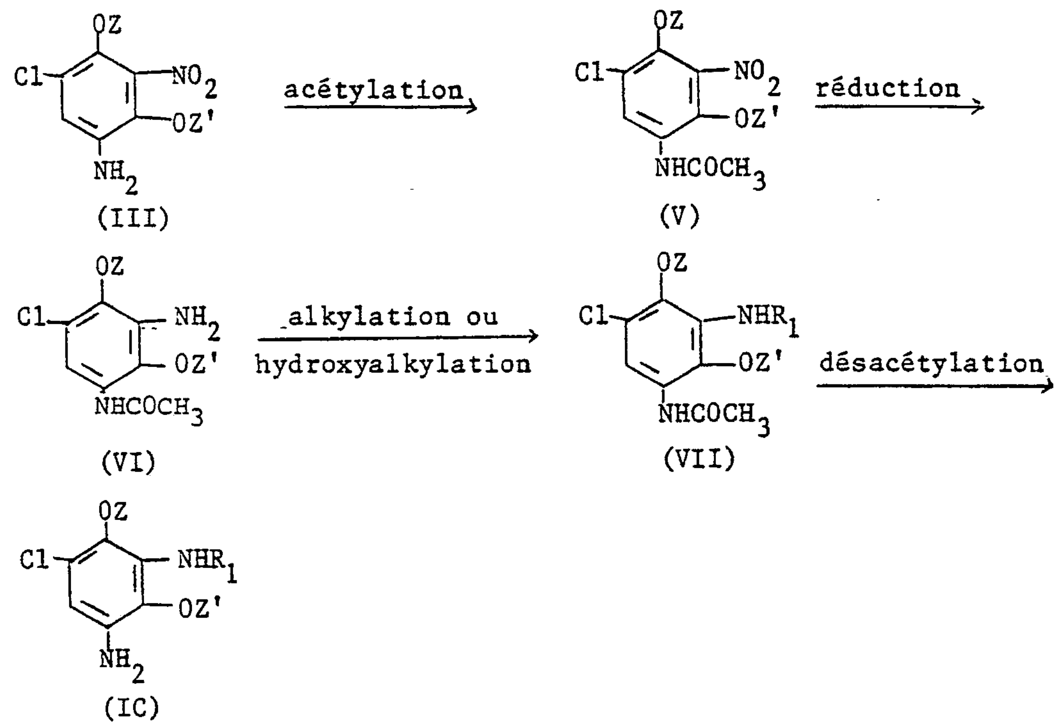

(III) (V) (VI) (VII) (IC)

Z, Z′ et $R_1$ ont les significations indiquées dans la formule (I).

Le composé de formule (III), préparé de la même façon que selon le procédé de préparation 3°), est acétylé par l'anhydride acétique à une température comprise entre 20° et 100° C. On obtient le composé (V).

Le composé (V) est réduit en composé (VI) en milieu acide acétique par le fer, à une température comprise entre 50 et 100° C.

Le composé (VI) est ensuite alkylé ou hydroxyalkylé selon des procédés connus pour conduire au composé (VII).

Celui-ci est désacétylé et on obtient ainsi le composé de formule (I) ou (I′) dans laquelle $R_1$ est différent de H et $R_2$ = H.

5°) Procédé de préparation des composés (I) ou (I′) dans lesquels $R_1 \neq R_2 \neq H$

Les composés (I) ou (I′) dans lesquels $R_1$ et $R_2$ sont différents de H mais ont des significations différentes peuvent être préparés par alkylation ou hydroxyalkylation des composés (IB) ou (IC) ci-dessus selon des méthodes classiques.

Les étapes d'alkylation et d'hydroxyalkylation mentionnées dans les procédés de préparation ci-dessus sont connues.

A titre d'agents alkylants, on utilise des halogénures d'alkyle ou des sulfates de dialkyle.

Pour l'hydroxyalkylation, la méthode préférée consiste à faire réagir le chloroformiate de β-chloréthyle sur le composé portant la fonction amine libre, à transformer le carbamate intermédiaire en oxazolidone qui est ensuite hydrolysée.

Ce procédé est décrit dans la demande de brevet français n° 2 571 364.

Le carbamate intermédiaire de β-chloréthyle peut aussi être soumis directement à l'action d'une base minérale forte telle que la soude ou la potasse pour donner le composé (I) dans lequel $R_1$ ou $R_2$ est un radical β-hydroxyéthyle.

Les composés de départ de formule (II) peuvent être obtenus par l'un des procédés suivants.

Procédé 1

Ce procédé est décrit dans "Recueil T. Chimiques Pays-Bas", R40, p. 451-471.

Il consiste à nitrer le 1,2,4-trichlorobenzène par l'acide nitrique fumant en présence éventuellement d'acide sulfurique. On obtient le 1,2,4-trichloro-3,5-dinitrobenzène dont les atomes de chlore en positions 2 et 4 sont ensuite substitués par les groupements OZ et OZ′ par réaction avec l'alcoolate alcalin correspondant.

Ce procédé peut être schématisé de la façon suivante :

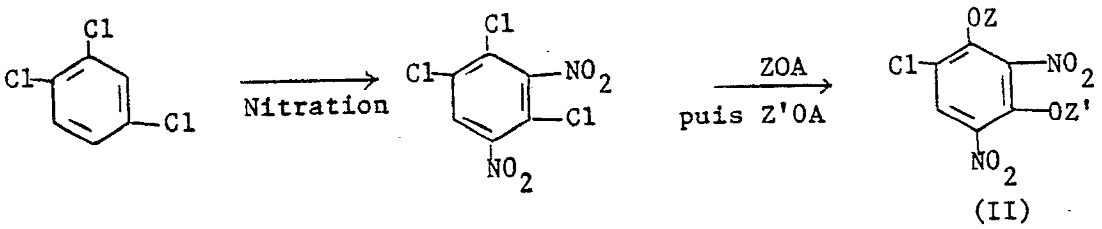


(II)

A étant un métal alcalin.

Procédé 2

Il consiste à nitrer le 2,4-dialcoxychlorobenzène ou le 2,4-di(hydroxyalcoxy)chlorobenzène par l'acide nitrique fumant en présence éventuellement d'acide sulfurique. On obtient, en une seule étape, respectivement, le 2,4-dialcoxy-3,5-dinitrochlorobenzène ou le 2,4-di(hydroxyalcoxy)-3,5-dinitrochlorobenzène.

Ce procédé peut être schématisé de la façon suivante :

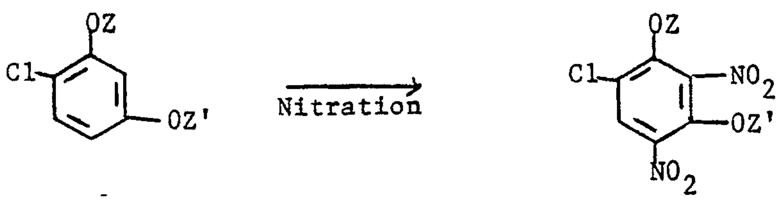


(II)

Procédé 3

Il consiste à alkyler ou à hydroxyalkyler le 3,6-dichlorophénol ou le 3,4-dichlorophénol, à nitrer le composé obtenu et enfin à substituer un atome de chlore par un groupement alcoxy ou hydroxyalcoxy par action de l'alcoolate alcalin correspondant.

Ce procédé est surtout intéressant dans le cas où Z et Z′ ne sont pas identiques.

Il peut être résumé par les deux schémas ci-après :

α)

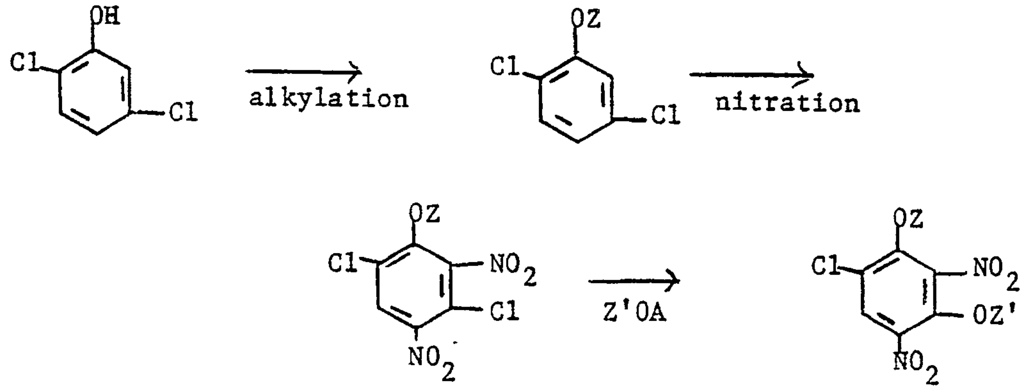


(II)

β)

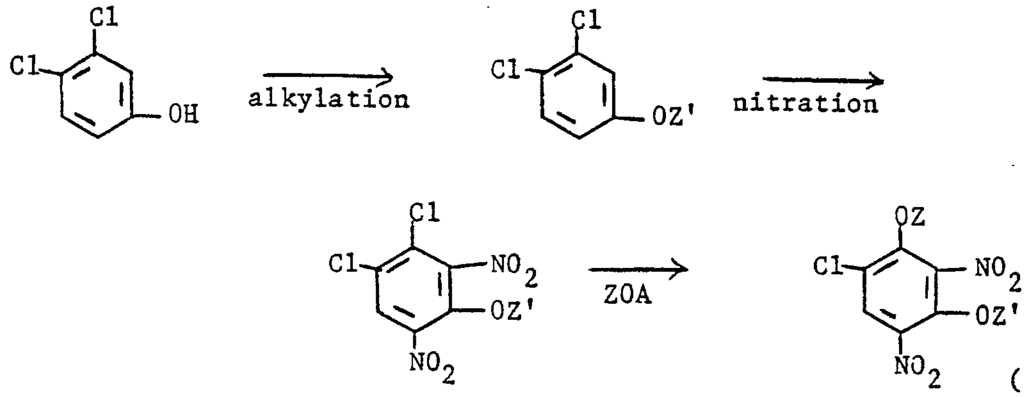


(II)

A étant un métal alcalin.

A titre de composés préférés de formule (I), on peut citer :

- le 5-(β-hydroxyéthyl)amino-3-amino-2,4-diméthoxychlorobenzène
- le 5-amino-3-(β-hydroxyéthyl)amino-2,4-diméthoxychlorobenzène
- le 3,5-di(β-hydroxyéthyl)amino-2,4-diméthoxychlorobenzène
- le 5-méthylamino-3-amino-2,4-diméthoxychlorobenzène
- le 3,5-diamino-2,4-diéthoxychlorobenzène
- le 3,5 diamino-2,4-di(γ-hydroxypropoxy)chlorobenzène

ainsi que leurs sels d'addition avec un acide et en particulier un acide minéral tel que l'acide chlorhydrique, l'acide bromhydrique ou l'acide sulfurique.

Les compositions tinctoriales pour fibres kératiniques et en particulier pour cheveux humains selon la présente invention contiennent au moins un composé de formule (I') ou l'un de ses sels d'addition avec un acide à titre de coupleur et au moins un précurseur de colorant d'oxydation de type para dans un support aqueux cosmétiquement acceptable.

A titre de coupleurs particulièrement préférés de formule (I'), on peut citer :

- le 5-(β-hydroxyéthyl)amino-3-amino-2,4-diméthoxychlorobenzène
- le 5-amino-3-(β-hydroxyéthyl)amino-2,4-diméthoxychlorobenzène
- le 3,5-di(β-hydroxyéthyl)amino-2,4-diméthoxychlorobenzène
- le 5-méthylamino-3-amino-2,4-diméthoxychlorobenzène
- le 3,5-diamino-2,4-diméthoxychlorobenzène
- le 3,5-diamino-2,4-diéthoxychlorobenzène
- le 3,5-diamino-2,4-di(γ-hydroxypropoxy)chlorobenzène

ainsi que leurs sels d'addition avec un acide et en particulier un acide minéral tel que l'acide chlorhydrique, bromhydrique ou sulfurique.

La demanderesse a constaté d'une manière surprenante, que le couplage des paraphénylènediamines avec les coupleurs de formule (I') dans laquelle $R_2$ est différent d'un atome d'hydrogène, en milieu oxydant, conduisait sur cheveux à des couleurs pourpres plus ou moins rouges. Le couplage des paraphénylènediamines avec les coupleurs de formule (I') où $R_2$ représente un atome d'hydrogène conduit à des nuances beiges qui sont utiles pour un formulaire de couleurs claires ou pour "rabattre" certains rouges.

Les composés de formule (I') ou leurs sels, utilisés avec des paraphénylènediamines, conduisent à des

nuances stables à la lumière, aux intempéries et au lavage, après développement en présence d'oxydant.

Le précurseur de colorant d'oxydation est choisi parmi les dérivés benzéniques ou hétérocycliques comme par exemple la pyridine sur lesquels sont fixés en position para deux groupements amino ou un groupement amino et un groupement hydroxy. Ces précurseurs de colorants peuvent être présents dans les compositions tinctoriales sous forme de bases libres ou sous forme de sels d'addition d'acides.

Les précurseurs de colorants d'oxydation particulièrement préférés utilisables conformément à l'invention, sont choisis parmi les paraphénylènediamines répondant à la formule générale suivante :

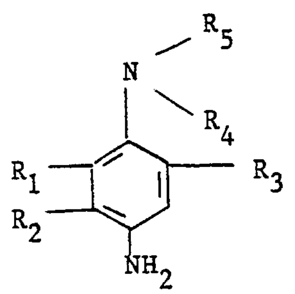

(VIII)

ou les sels correspondants, formule dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ peuvent former conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène.

Parmi les composés de formule (VIII) indiquée ci-dessus, on peut citer la p-phénylènediamine, la p-toluylènediamine, la méthoxyparaphénylènediamine, la chloroparaphénylènediamine, la 2,6-diméthylparaphénylènediamine, la 2,5-diméthyl-paraphénylènediamine, l'isopropyl-p-phénylènediamine, la 2,3-diméthyl-paraphénylènediamine, la 2-méthyl-5-méthoxy-paraphénylènediamine, la 2,6-diméthyl-5-méthoxyparaphénylènediamine, la N,N-diméthylparaphénylènediamine, la 3-méthyl-4-amino-N,N-diéthylaniline, la N,N-di-(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl) aniline, la 3-méthyl-4-amino-N,N-(éthyl, carbamylméthyl) aniline, la 4-amino-N,N-(éthyl, β-pipéridinoéthyl)aniline, la 3-méthyl-4- amino-N,N-(éthyl, β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)aniline, la 4-amino-N,β-méthoxyéthylaniline, la 3-méthyl-4-amino-N,N-(éthyl, β-acétylaminoéthyl)aniline, la 4-amino-N,N-(éthyl, β-mésylaminoéthyl)aniline, la 3-méthyl-4-amino-N,N-(éthyl, β-mésylaminoéthyl)aniline, la 4-amino-N,N-(éthyl, β-sulfoéthyl)aniline, la 3-méthyl-4-amino-N,N-(éthyl, β-sulfoéthyl)aniline, la N-[(4′-amino)phényl]morpholine, la N-[(4′-amino)phényl] pipéridine. Ces précurseurs de colorants par oxydation de type para peuvent être introduits dans la composition tinctoriale sous forme de base libre ou sous forme de sels, tels que sous forme de chlorhydrate, de bromhydrate ou de sulfate.

Le composé de formule (I′) ou ses sels peut également être utilisé avec des para-aminophénols pour donner des nuances brun clair à jaune stables à la lumière, aux intempéries et au lavage après développement en présence d'oxydant. Parmi les para-aminophénols, on peut citer le para-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

Le composé de formule (I′) ou ses sels peut également être utilisé avec des précurseurs de colorants d'oxydation para hétérocycliques parmi lesquels on peut citer la 2,5-diaminopyridine, la 2-hydroxy-5-aminopyridine, la tétraaminopyrimidine.

Les compositions tinctoriales selon l'invention peuvent également contenir des précurseurs de colorants d'oxydation de type ortho tels que les orthoaminophénols, orthophénylènediamines, orthodiphénols. On peut citer par exemple le 1-amino2-hydroxybenzène, le 6-méthyl-1-hydroxy 2-aminobenzène, le 4-méthyl-1-amino-2-hydroxybenzène.

Les compositions tinctoriales conformes à l'invention contenant le composé (I′) ou ses sels peuvent contenir éventuellement d'autres coupleurs connus en eux-mêmes tels que les métadiphénols, les métaaminophénols, les métaphénylènediamines, les métaacylaminophénols, les métauréidophénols, les métacarbalcoxyaminophénols, l'α-naphtol, les coupleurs possédant un groupe méthylène actif tels que les composés β-cétoniques et les pyrazolones.

On peut citer en particulier, à titre d'exemple, le 2,4-dihydroxyphénoxyéthanol,le 2,4-dihydroxyanisole, le métaaminophénol, le monométhyléther de résorcine, le 2-méthyl-5-aminophénol, le 2-méthyl-5-N-(β-hydroxyéthyl)aminophénol, le 2-méthyl-5-N-(β-mésylaminoéthyl)aminophénol, le 2,6-diméthyl-3-aminophénol, la 6-hydroxybenzomorpholine, le 2,4-diaminophénoxyéthanol, la 6-aminobenzomorpholine, le 2-[N-(β-hydroxyéthyl)amino]-4-aminophénoxyéthanol, le 2-amino-4-N-(β-hydroxyéthyl)aminoanisole, le (2,4-diamino)phényl-β,γ-dihydroxypropyléther, la 2,4-diaminophénoxyéthylamine, le 3,4-méthylènedioxyphénol, la 3,4-méthylènedioxyaniline, et leurs sels.

On peut rajouter à ces compositions, comme cela est bien connu en vue de nuancer ou enrichir en reflets les colorations apportées par les précurseurs de colorants d'oxydation, des colorants directs tels que des colorants azoïques, anthraquinoniques ou les dérivés nitrés de la série benzénique.

L'ensemble des composés para et des coupleurs utilisés dans les compositions tinctoriales conformes à l'invention représente de préférence de 0,1 à 7% en poids de ladite composition. La concentration en composé (I′) peut varier entre 0,05 et 3,5% du poids total de la composition.

Le milieu aqueux cosmétiquement acceptable a un pH qui peut varier entre 8 et 11 et il est de préférence compris entre 9 et 11.

Il est ajusté à la valeur désirée à l'aide d'un agent alcalinisant tel que l'ammoniaque, les carbonates alcalins, les alcanolamines comme la mono-, la di- ou la triéthanolamine.

Les compositions tinctoriales conformes à l'invention contiennent également, dans leur forme de réalisation préférée, des agents tensio-actifs anioniques, cationiques, non-ioniques amphotères ou leurs mélanges. Parmi ces agents tensio-actifs, on peut citer plus particulièrement les alkylbenzènesulfonates, les alkylnaphtalènesulfonates, les sulfates, les éther-sulfates et sulfonates d'alcools gras, les sels d'ammonium quaternaire tels que le bromure de triméthylcétylammonium, le bromure de cétylpyridinium, les éthanolamides d'acides gras éventuellement oxyéthylénés, les acides, les alcools et les amines polyoxyéthylénés, les alcools polyglycérolés, les alkylphénols polyoxyéthylénés ou polyglycérolés ainsi que les alkylsulfates polyoxyéthylénés. Les agents tensio-actifs sont présents dans les compositions conformes à l'invention dans des proportions comprises entre 0,5 et 40% en poids, et de préférence entre 4 et 30% en poids par rapport au poids total de la composition.

Ces compositions peuvent également contenir des solvants organiques pour solubiliser les composés qui ne seraient pas suffisamment solubles dans l'eau. Parmi ces solvants, on peut mentionner à titre d'exemple, les alcanols inférieurs en $C_1$-$C_4$ tels que l'éthanol et l'isopropanol; le glycérol; les glycols ou éthers de glycol comme le 2-butoxyéthanol, l'éthylèneglycol, le propylèneglycol, le monoéthyléther et le monométhyléther du diéthylèneglycol, ainsi que les produits analogues et leurs mélanges. Les solvants sont présents de préférence dans une proportion comprise entre 1 et 40% en poids, et en particulier entre 5 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose tels que la méthylcellulose, l'hydroxyéthylcellulose, l'hydroxypropylcellulose, l'hydroxyméthylcellulose, la carboxyméthylcellulose, les polymères d'acide acrylique, la gomme de Xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids par rapport au poids total de la composition.

Les compositions peuvent contenir des agents anti-oxydants choisis en particulier parmi le sulfite de sodium, l'acide thioglycolique, le bisulfite de sodium, l'acide ascorbique et l'hydroquinone. Ces agents anti-oxydants sont présents dans la composition dans des proportions comprises entre 0,05 et 1,5% en poids par rapport au poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses telles que sous forme de liquides, de crèmes, de gels ou sous toute autre forme appropriée pour réaliser une teinture des fibres kératiniques et notamment des cheveux humains. Elles peuvent également être conditionnées en flacons aérosols en présence d'un agent propulseur.

Les compositions tinctoriales conformes à l'invention contenant un précurseur de colorant par oxydation de type para et le composé (I′) ou l'un de ses sels sont utilisées dans un procédé de teinture capillaire

mettant en oeuvre la révélation par un oxydant.

Conformément à ce procédé, on mélange au moment de l'emploi la composition tinctoriale décrite ci-dessus avec une solution oxydante en une quantité suffisante, puis on applique le mélange obtenu sur les cheveux.

La solution oxydante contient des agents d'oxydation tels que l'eau oxygénée, le peroxyde d'urée ou les persels tels que le persulfate d'ammonium. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Un autre procédé de mise en oeuvre du composé (I′) conforme à l'invention consiste à teindre les cheveux suivant un procédé en plusieurs temps, selon lequel, dans un premier temps, on applique le précurseur de colorant d'oxydation para au moyen d'une composition sus-définie et dans un second temps, on applique le composé (I′). L'agent oxydant est présent dans la composition appliquée dans le second temps ou bien appliqué sur les cheveux eux-mêmes dans un troisième temps, les conditions de pose, de séchage et lavage étant identiques à celles indiquées au procédé ci-dessus.

Les exemples ci-après servent à mieux illustrer l'invention mais ne sont en aucun cas limitatifs de sa portée.

Exemple de préparation 1

Préparation du dichlorhydrate de 5-(β-hydroxyéthyl) amino-3-amino-2,4-diméthoxychlorobenzène

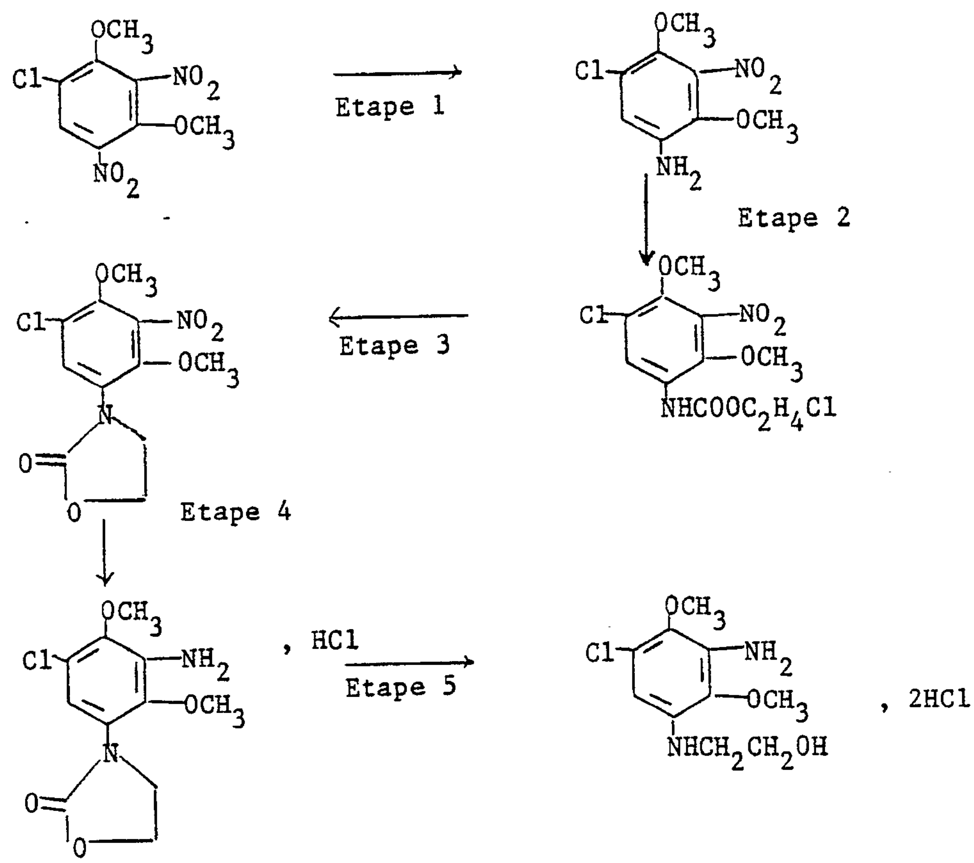


Etape 1

Préparation du 5-amino-2,4-diméthoxy-3-nitrochlorobenzène

On porte au reflux pendant 1 heure le mélange constitué de 0,2 mole (52,5 g) de 3,5-dinitro-2,4-diméthoxychlorobenzène, de 15,9 g de palladium sur charbon (10%) dans 260 ml d'éthanol absolu additionnés de 110 ml de cyclohexène. Le catalyseur est éliminé par filtration. Par évaporation à sec sous

vide, on obtient une huile qui cristallise par ajout d'eau glacée. Après essorage et séchage, le produit obtenu est recristallisé d'un mélange de benzène et de cyclohexane. Il fond à 68° C.

L'analyse du produit obtenu donne les résultats suivants :

| | Calculé pour $C_8H_9N_2O_4Cl$ | Trouvé |
|---|---|---|
| %C | 41,28 | 41,10 |
| %H | 3,87 | 3,90 |
| %N | 12,04 | 11,98 |
| %O | 27,52 | 27,68 |
| %Cl | 15,27 | 14,99 |

Etape 2

Préparation du 5-(β-chloréthoxycarbonyl)amino-2,4-diméthoxy-3-nitrochlorobenzène

On dissout 0,05 mole (11,7 g) de 5-amino-2,4-diméthoxy-3-nitrochlorobenzène dans 50 ml de dioxane. On ajoute 5 g de carbonate de calcium puis on élève la température au voisinage de 90° C. On introduit alors sous agitation 0,05 mole (7,2 g) de chloroformiate de β-chloréthyle. L'addition terminée, on maintient l'agitation 30 minutes supplémentaires à 90° C. Les sels minéraux présents dans le milieu réactionnel sont éliminés par filtration à chaud. Après addition d'eau glacée au filtrat, le produit attendu cristallise. Le produit obtenu est essoré, lavé à l'eau. Après séchage, il est recristallisé de l'éthanol. Il fond à 93° C.

L'analyse du produit obtenu donne les résultats suivants :

| | Calculé pour $C_{11}H_{12}Cl_2N_2O_6$ | Trouvé |
|---|---|---|
| %C | 38,94 | 38,78 |
| %H | 3,54 | 3,53 |
| %N | 8,26 | 8,35 |
| %O | 28,32 | 28,40 |
| %Cl | 20,94 | 21,03 |

Etape 3

Préparation de la N-[(3′-chloro-4′,6′-diméthoxy-5′-nitro)phényl]oxazolidine-1,3 one-2

On chauffe à 70° C 0,03 mole (10,2 g) de carbamate de β-chloréthyle obtenu selon le mode opératoire décrit à l'étape 2 dans 50 ml de méthanol. On ajoute rapidement 0,03 mole de méthylate de sodium en solution à 30% dans le méthanol. Le chauffage est maintenu 15 minutes supplémentaires après la fin de l'addition. Les sels minéraux sont éliminés par filtration. Du filtrat refroidi et dilué à l'eau glacée on isole par filtration le produit attendu qui a cristallisé. Après séchage, il est recristallisé de l'éthanol. Il fond à 133° C.

L'analyse du produit obtenu donne les résultats suivants :

| | Calculé pour $C_{11}H_{11}ClN_2O_6$ | Trouvé |
|---|---|---|
| %C | 43,64 | 43,81 |
| %H | 3,64 | 3,64 |
| %N | 9,26 | 9,37 |
| %O | 31,74 | 31,62 |
| %Cl | 11,74 | 11,74 |

Etape 4
Préparation du chlorhydrate de la N-[(3'-chloro-4',6'-diméthoxy-5'-amino)phényl]oxazolidine-1,3 one-2

A 60 ml d'eau additionnés de 3 ml d'acide acétique et préalablement chauffés au bain-marie bouillant, on ajoute 12 g de fer en poudre réduit à l'hydrogène et peu à peu, sous agitation, 0,02 mole (6,05 g) de l'oxazolidine-1,3 one-2 obtenue selon le mode opératoire décrit à l'étape précédente. Les additions terminées, on maintient le chauffage pendant 15 minutes supplémentaires. Les boues ferriques sont éliminées du milieu réactionnel par filtration à chaud. Le milieu réactionnel débarrassé des boues ferriques est extrait à l'acétate d'éthyle. La phase acétate d'éthyle est lavée à l'eau et séchée sur sulfate de sodium. Par ajout d'une solution 7N d'acide chlorhydrique dans l'éthanol absolu, on précipite le produit attendu.

L'analyse du produit obtenu donne les résultats suivants :

| | Calculé pour $C_{11}H_{14}N_2O_4Cl_2$ | Trouvé |
|---|---|---|
| %C | 42,72 | 42,59 |
| %H | 4,53 | 4,64 |
| %N | 9,06 | 8,96 |
| %O | 20,71 | 20,60 |
| %Cl | 22,98 | 22,87 |

Etape 5
Préparation du dichlorhydrate de 5-(β-hydroxyéthyl)amino-3-amino-2,4-diméthoxychlorobenzène

On ajoute 0,03 mole (9,3 g) de chlorhydrate de la N-[(3'-chloro-4',6'-diméthoxy-5'-amino)phényl]-oxazolidine-1,3 one-2 obtenu selon le mode opératoire décrit dans l'étape précédente à 10 ml d'eau additionnés de 10 ml d'éthanol. A cette solution on ajoute 18 ml de soude 10N. On chauffe 30 minutes à 80°C. Après refroidissement et décantation, la phase supérieure est diluée à l'acétate d'éthyle. Après lavage à l'eau puis séchage sur sulfate de sodium, on ajoute 14 ml d'une solution d'acide chlorhydrique 7N dans l'éthanol absolu. Le produit attendu précipite. Il est recristallisé d'une solution hydroalcoolique d'acide chlorhydrique.

L'analyse du produit obtenu donne les résultats suivants :

| | Calculé pour $C_{10}H_{17}N_2O_3Cl_3$ | Trouvé |
|---|---|---|
| %C | 37,56 | 37,44 |
| %H | 5,32 | 5,24 |
| %N | 8,76 | 8,83 |
| %O | 15,02 | 15,21 |
| %Cl | 33,33 | 33,07 |

Exemple de préparation 2
Préparation du dichlorhydrate de 5-amino-3-(β-hydroxyéthyl)amino 2,4-diméthoxychlorobenzène

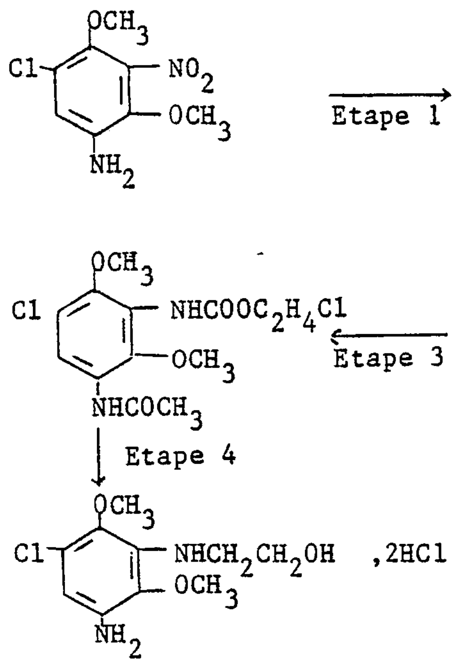


Etape 1

Préparation du 5-acétamido-2,4-diméthoxy-3-nitrochlorobenzène

On chauffe à 70° C 330 ml d'anhydride acétique additionnés de quelques gouttes d'acide sulfurique. On ajoute sous agitation 0,7mole (163 g) de 5-amino-2,4-diméthoxy-3-nitrochlorobenzène préparé selon le mode opératoire décrit dans l'exemple de préparation 1 (étape 1).

Par dilution du milieu réactionnel par de l'eau glacée le produit attendu précipite. Recristallisé de l'éthanol à 96°, il fond à 115° C.

L'analyse du produit obtenu donne les résultats suivants :

| | Calculé pour $C_{10}H_{11}N_2O_5Cl$ | Trouvé |
|---|---|---|
| %C | 43,72 | 43,67 |
| %H | 4,01 | 4,07 |
| %N | 10,20 | 10,17 |
| %O | 29,14 | 29,04 |
| %Cl | 12,93 | 12,98 |

Etape 2

Préparation du 5-acétamido-2,4-diméthoxy-3-aminochlorobenzène

A 760 ml d'eau additionnés de 13 ml d'acide acétique et préalablement chauffés à 80° C, on ajoute 253 g de fer en poudre réduit à l'hydrogène et peu à peu, sous agitation, 0,46 mole (126,5 g) de 5-acétamido-2,4-diméthoxy-3-nitro chlorobenzène préparé à l'étape précédente. Les additions terminées, on maintient le milieu réactionnel au bain-marie bouillant pendant 15 minutes. Après refroidissement, le milieu réactionnel est centrifugé. Les boues ferriques séparées de la phase liquide sont extraites à l'acétate d'éthyle. La phase acétate d'éthyle, lavée à l'eau puis séchée sur sulfate de sodium, est diluée par 100 ml d'une solution 7N d'acide chlorhydrique dans l'éthanol absolu. Le produit attendu, qui a précipité sous forme de chlorhydrate, est essoré. Il est dissous dans le minimum d'eau. Après neutralisation, le produit attendu précipite. Recristallisé de l'alcool, il fond à 113° C.

L'analyse du produit obtenu donne les résultats suivants :

| | Calculé pour $C_{10}H_{13}ClN_2O_3$ | Trouvé |
|---|---|---|
| %C | 49,08 | 49,12 |
| %H | 5,32 | 5,31 |
| %N | 11,45 | 11,50 |
| %O | 19,63 | 19,74 |
| %Cl | 14,52 | 14,49 |

Etape 3

Préparation du 5-acétamido- 3-(β-chloréthoxycarbonyl)amino-2,4-diméthoxychlorobenzène

A 70 ml de dioxane on ajoute 0,05 mole (14 g) de chlorhydrate de 5-acétamido-3-amino-2,4-diméthoxychlorobenzène obtenu selon l'étape précédente, puis 5,5 ml de soude 10N. On élève la température au voisinage de 90° C puis on ajoute 5 g de carbonate de calcium. On introduit alors sous agitation 7,55 g de chloroformiate de β-chloréthyle. L'addition terminée, on maintient le chauffage 30 minutes à 90° C. Le milieu réactionnel est dilué par un mélange glace/eau après refroidissement. Le produit attendu précipite après acidification du milieu réactionnel. Recristallisé de l'éthanol, il fond à 148° C.

L'analyse du produit obtenu donne les résultats suivants :

| | Calculé pour $C_{13}H_{16}Cl_2N_2O_5$ | Trouvé |
|---|---|---|
| %C | 44,44 | 44,46 |
| %H | 4,56 | 4,54 |
| %N | 7,98 | 7,95 |
| %O | 22,79 | 22,66 |
| %Cl | 20,23 | 20,29 |

Etape 4

Préparation du dichlorhydrate de 5-amino-3-(β-hydroxyéthyl)amino-2,4-diméthoxychlorobenzène

On porte au reflux le mélange constitué de 0,25 mole (88 g) de carbamate de β-chloréthyle obtenu à l'étape précédente et de 250ml de soude 10N dans 10 ml d'eau additionnés de 15 ml d'éthanol. Après 1 heure de chauffage le milieu réactionnel refroidi et neutralisé est extrait à l'acétate d'éthyle. Les phases acétate d'éthyle rassemblées sont évaporées sous vide après avoir été lavées et séchées sur sulfate de sodium. L'extrait sec obtenu est dissous dans l'éther isopropylique. Le produit attendu précipite après ajout d'une solution 7N d'acide chlorhydrique dans l'éthanol absolu. Il est recristallisé d'un mélange hydroalcoolique contenant de l'acide chlorhydrique.

L'analyse du produit obtenu donne les résultats suivants :

| | Calculé pour $C_{10}H_{17}N_2O_3Cl_3$ | Trouvé |
|---|---|---|
| %C | 37,56 | 37,59 |
| %H | 5,32 | 5,36 |
| %N | 8,76 | 8,68 |
| %O | 15,02 | 15,24 |
| %Cl | 33,33 | 33,21 |

Exemple de préparation 3

Préparation du dichlorhydrate de 3,5-diamino-2,4-diméthoxychlorobenzène

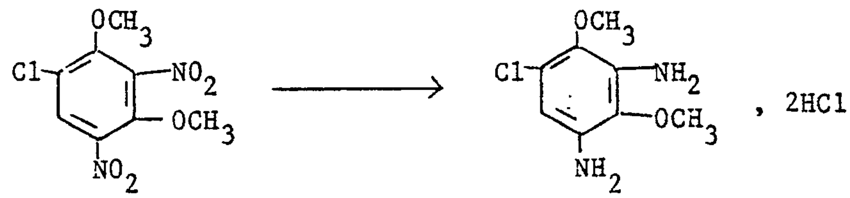

A 270 ml d'eau additionnés de 27 ml d'acide acétique et préalablement chauffés à 80 °C, on ajoute 100 g de fer en poudre réduit à l'hydrogène et peu à peu, sous agitation, 0,25 mole (66 g) de 3,5-dinitro-2,4-diméthoxychlorobenzène. Les additions terminées, on maintient le milieu réactionnel au bain-marie bouillant pendant 30 minutes supplémentaires. Après refroidissement, le milieu réactionnel est centrifugé. Les boues ferriques qui contiennent le produit attendu sont reprises sous brassage par de l'acétone. Après filtration des boues ferriques puis lavage à l'acétone, le produit attendu précipite du filtrat acétonique par ajout d'une solution d'acide chlorhydrique dans l'éthanol. Après essorage et lavage, le produit attendu est recristallisé à chaud d'un mélange d'acide chlorhydrique et d'eau.

L'analyse du produit obtenu donne les résultats suivants :

| | Calculé pour $C_8H_{13}N_2Cl_3O_2$ | Trouvé |
|---|---|---|
| %C | 34,85 | 34,85 |
| %H | 4,72 | 4,82 |
| %N | 10,16 | 10,03 |
| %O | 11,62 | 11,80 |
| %Cl | 38,66 | 38,46 |

Exemple de préparation 4

Préparation du dichlorhydrate de 3,5-di (β-hydroxyéthyl)amino-2,4-diméthoxychlorobenzène

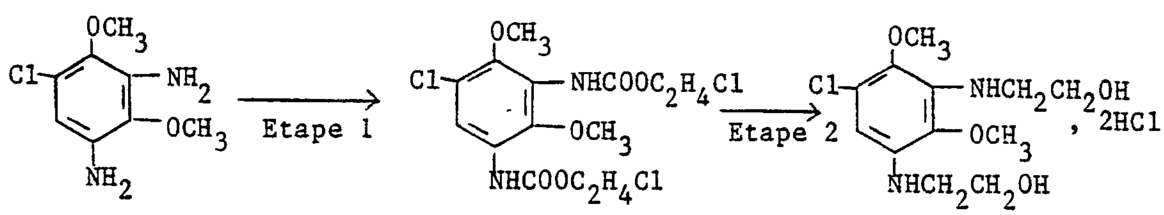


Etape 1

Préparation du 3,5-di-(β-chloréthoxycarbonyl)amino-2,4-diméthoxychlorobenzène

A 200 ml de dioxane, on ajoute 0,15 mole (41,3 g) de dichlorhydrate de 3,5-diamino-2,4-diméthoxychlorobenzène obtenu à l'exemple 3, puis 30 ml de soude 10N. On élève la température au voisinage de 80 °C, puis on ajoute 15 g de carbonate de calcium. On introduit alors sous agitation 43 g de chloroformiate de β-chloréthyle. L'addition terminée, on maintient le chauffage à 90 °C pendant 15 minutes. Les sels minéraux sont éliminés du milieu réactionnel par filtration. Le produit attendu cristallise lentement après dilution du milieu réactionnel par de l'eau glacée. Recristallisé de l'éthanol à 96°, il fond à 87 °C.

L'analyse du produit obtenu donne les résultats suivants :

| | Calculé pour $C_{14}H_{17}N_2O_6Cl_3$ | Trouvé |
|---|---|---|
| %C | 40,43 | 40,41 |
| %H | 4,09 | 4,08 |
| %N | 6,74 | 6,82 |
| %O | 23,10 | 23,06 |
| %Cl | 25,63 | 25,51 |

Etape 2

Préparation du dichlorhydrate de 3,5-di-(β-hydroxyéthyl)amino-2,4-diméthoxychlorobenzène

A 72 ml d'éthanol on ajoute 0,08 mole (33,3 g) du composé préparé à l'étape précédente puis 72 ml de soude 10N diluée par 36 ml d'eau. Après 30 minutes de chauffage au reflux, l'éthanol est éliminé du milieu réactionnel par distillation sous pression réduite. Le milieu réactionnel est neutralisé puis extrait à l'acétate d'éthyle. Les phases acétate d'éthyle sont lavées à l'eau. On ajoute 30 ml d'une solution 7N d'acide chlorhydrique dans l'éthanol absolu. Le produit attendu précipite lentement sous forme d'hémi-hydrate. Il est recristallisé de l'éthanol.

L'analyse du produit obtenu donne les résultats suivants :

| | Calculé pour $C_{12}H_{21}N_2O_4Cl_3$ $1/_2H_2O$ | Trouvé |
|---|---|---|
| %C | 38,65 | 38,55 |
| %H | 6,17 | 5,84 |
| %N | 7,59 | 7,43 |
| %O | 19,32 | 19,28 |
| %Cl | 28,59 | 28,50 |

Exemple de préparation 5

Préparation du dichlorhydrate de 5-méthylamino-3-amino-2,4-diméthoxychlorobenzène

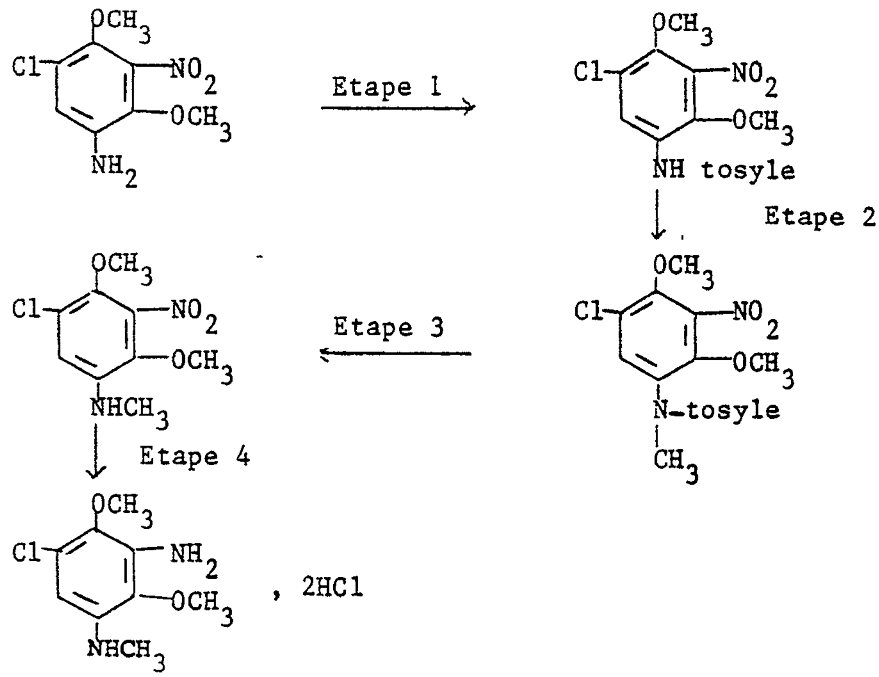

Etape 1
Préparation du 5-N-tosylamino-2,4-diméthoxy-3-nitro-chlorobenzène

On ajoute peu à peu à 40°C 0,11 mole (21 g) de p-toluènesulfochlorure à une solution de 0,1 mole (23,35 g) de 5-amino-2,4-diméthoxy-3-nitrochlorobenzène. On poursuit l'agitation 15 minutes à 40°C après la fin de l'addition. Le mélange réactionnel est dilué par de l'eau glacée. Par acidification par l'acide chlorhydrique concentré, le produit attendu précipite. Après essorage, lavage à l'eau puis à l'alcool, le produit est séché. Il est recristallisé de l'éthanol.

L'analyse du produit obtenu donne les résultats suivants :

| Analyse | Calculé pour $C_{15}H_{15}N_2O_6SCl$ | Trouvé |
|---|---|---|
| C% | 46,57 | 46,49 |
| H% | 3,88 | 3,90 |
| N% | 7,24 | 7,38 |
| O% | 24,84 | 24,56 |
| S% | 8,28 | 8,17 |
| Cl% | 9,18 | 9,35 |

Etape 2
Préparation du 5-N,N-tosyl,méthylamino-2,4-diméthoxy-3-nitrochlorobenzène

On ajoute à 30°-35°C 0,022 mole (2,1 ml) de sulfate de méthyle à une solution de 0,02 mole (7,7g) de 5-N-tosylamino-2,4-diméthoxy-3-nitrochlorobenzène préparé à l'étape précédente dans 25 ml d'une solution normale de soude. On poursuit l'agitation 15 minutes après la fin de l'addition. Le produit attendu précipite par dilution du milieu réactionnel avec de l'eau glacée. Après essorage et lavage à l'eau, le produit obtenu est recristallisé de l'alcool.

L'analyse du produit obtenu donne les résultats suivants :

| Analyse | Calculé pour $C_{16}H_{17}N_2O_6SCl$ | Trouvé |
|---|---|---|
| C% | 47,94 | 47,96 |
| H% | 4,24 | 4,28 |
| N% | 6,99 | 7,00 |
| O% | 23,97 | 23,94 |
| S% | 7,99 | 7,84 |
| Cl% | 8,86 | 8,91 |

Etape 3

Préparation du 5-méthylamino-2,4-diméthoxy-3-nitrochlorobenzène

On ajoute peu à peu 0,025 mole (10 g) du composé préparé selon l'étape précédente à 20 ml d'acide sulfurique concentré, la température étant maintenue à 20° C. 15 minutes après la fin de l'addition, le milieu réactionnel est dilué par un mélange glace/eau. Le produit attendu, qui a précipité, est essoré, lavé à l'eau, séché sous vide en présence de $P_2O_5$. Il est recristallisé du cyclohexane. Il fond à 57° C. L'analyse du produit obtenu donne les résultats suivants :

| Analyse | Calculé pour $C_9H_{11}N_2O_4Cl$ | Trouvé |
|---|---|---|
| C% | 43,81 | 43,77 |
| H% | 4,46 | 4,55 |
| N% | 11,36 | 11,35 |
| O% | 25,96 | 26,12 |
| Cl | 14,40 | 14,36 |

Etape 4

Préparation du dichlorhydrate de 3-amino-5-méthylamino-2,4-diméthoxychlorobenzène

A 200 ml d'eau additionnés de 2,5 ml d'acide acétique et préalablement chauffés au bain-marie bouillant, on ajoute 50 g de fer en poudre réduit à l'hydrogène et peu à peu, sous agitation, 0,1 mole (24,65 g) de 5-méthylamino-2,4-diméthoxy-3-nitrochlorobenzène obtenu selon le mode opératoire de l'étape 3. Les additions terminées, on maintient le chauffage 15 minutes supplémentaires. Le milieu réactionnel est centrifugé, le produit attendu est extrait des boues ferriques par l'acétate d'éthyle. Les phases acétate d'éthyle sont lavées à l'eau, puis séchées sur sulfate de sodium anhydre. Par ajout de 43 ml d'une solution 7N d'acide chlorhydrique dans l'éthanol, on précipite le produit attendu. Après essorage du précipité, lavage à l'éthanol puis séchage, le produit est recristallisé d'une solution hydroalcoolique d'acide chlorhydrique.

L'analyse du produit obtenu donne les résultats suivants :

| Analyse | Calculé pour $C_9H_{15}N_2O_2Cl_3$ | Trouvé |
|---|---|---|
| C% | 37,31 | 37,21 |
| H% | 5,18 | 5,23 |
| N% | 9,67 | 9,54 |
| O% | 11,05 | 11,10 |
| Cl% | 36,79 | 36,86 |

Exemple de préparation 6

Préparation du dichlorhydrate de 3,5-diamino-2,4-diéthoxy chlorobenzène

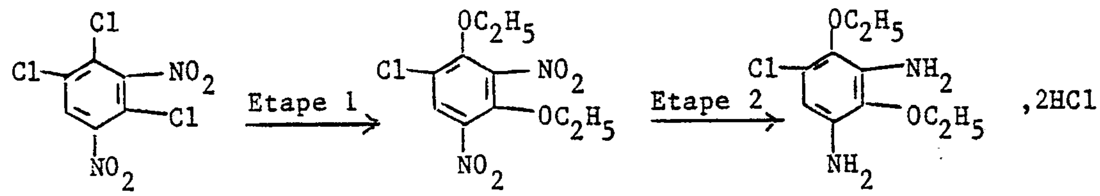

Etape 1

Préparation du 3,5 -dinitro-2,4-diéthoxychlorobenzène

On porte à 75° C 0,1 mole (27,15 g) de 3,5-dinitro-1,2,4-trichlorobenzène dans 110 ml d'éthanol absolu. On ajoute 0,2 mole d'éthylate de sodium en solution à 15% dans l'éthanol absolu. Le mélange réactionnel est chauffé 30 minutes à 75° C après la fin de la coulée, puis dilué par 300 g d'un mélange glace/eau. Le produit attendu précipite. Après séchage sous vide et recristallisation de l'éther isopropylique, il fond à 78° C.

L'analyse du produit obtenu est la suivante :

| Analyse | Calculé pour $C_{10}H_{11}N_2O_6Cl$ | Trouvé |
|---|---|---|
| C% | 41,31 | 41,22 |
| H% | 3,79 | 3,81 |
| N% | 9,64 | 9,65 |
| O% | 33,05 | 32,88 |
| Cl% | 12,22 | 12,07 |

Etape 2

Préparation du dichlorhydrate de 3,5-diamino-2,4-diéthoxy chlorobenzène

A 450 ml d'eau additionnés de 8,5 ml d'acide acétique préalablement chauffé à 80° C, on ajoute 170 g de fer en poudre réduit à l'hydrogène et peu à peu, sous agitation, 0,3 mole (87 g) de 3,5-dinitro-2,4-diéthoxychlorobenzène. Les additions terminées, on maintient le milieu réactionnel au bain-marie bouillant pendant 30 minutes supplémentaires. Après refroidissement, le milieu réactionnel est centrifugé. Les boues ferriques sont réempatées dans l'acétate d'éthyle, et les eaux mères sont extraites à l'acétate d'éthyle. Les phases acétate d'éthyle réunies sont lavées à l'eau, puis séchées sur $Na_2SO_4$. Par ajout d'une solution d'acide chlorhydrique dans l'éthanol, le produit attendu précipite. Après essorage, il est lavé à l'acétone. Il est recristallisé d'une solution hydro-alcoolique d'acide chlorhydrique.

L'analyse du produit obtenu donne les résultats suivants :

| Analyse | Calculé pour $C_{10}H_{17}N_2O_2Cl_3$ | Trouvé |
|---|---|---|
| C% | 39,54 | 39,67 |
| H% | 5,60 | 5,64 |
| N% | 9,23 | 9,26 |
| O% | 10,54 | 10,48 |
| Cl% | 35,09 | 35,26 |

Exemple de préparation 7

Préparation du dichlorhydrate de 3,5-diamino-2,4-di-(γ-hydroxypropoxy)chlorobenzène

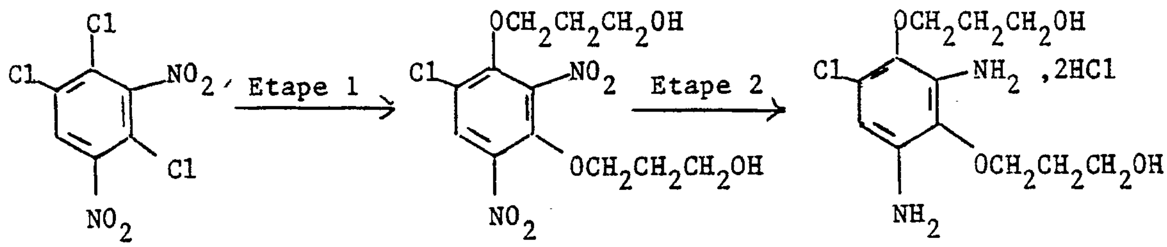

Etape 1

Préparation du 3,5-dinitro-2,4-di-(γ-hydroxypropoxy)chlorobenzène

On ajoute à 85 °C, 1 mole (271,5 g) de 3,5-dinitro-1,2,4-trichlorobenzène dans 700 ml de 1,3-propanediol. On ajoute en 30 minutes 2 moles de potasse en poudre dans 280 ml de 1,3-propanediol. Le mélange réactionnel est chauffé 1 heure à 85 °C après la fin de la l'addition. Après refroidissement, le produit attendu est filtré, lavé à l'eau, puis dissous dans 1,5 litre d'acétate d'éthyle qui, après lavage à l'eau, est séché sur sulfate de sodium. Après concentration à sec, on obtient un précipité qui, recristallisé de l'éther isopropylique, fond à 90 °C.

L'analyse du produit obtenu donne les résultats suivants :

| Analyse | Calculé pour $C_{12}H_{15}N_2O_8Cl$ | Trouvé |
|---|---|---|
| C% | 41,08 | 41,12 |
| H% | 4,28 | 4,33 |
| N% | 7,99 | 8,04 |
| O% | 36,52 | 36,45 |
| Cl% | 10,13 | 10,09 |

Etape 2

Préparation du dichlorhydrate de 3,5-diamino-2,4-di-(γ-hydroxypropoxy)chlorobenzène

A 700 ml d'eau additionnés de 10 ml d'acide acétique préalablement chauffé à 95 °C, on ajoute 250 g de fer en poudre réduit à l'hydrogène et peu à peu, sous agitation, 0,34 mole (119 g) de 3,5-dinitro-2,4-di-(γ-hydroxypropoxy)chlorobenzène. Les additions terminées, on maintient le milieu réactionnel 10 minutes supplémentaires à 95 °C. Après refroidissement, on ajoute 1 litre d'acétate d'éthyle. Le milieu réactionnel est filtré afin d'éliminer les boues ferriques. La phase acétate d'éthyle, séparée de la phase aqueuse, est, après lavage à l'eau puis séchage sur du sulfate de sodium, chassée à sec. L'extrait sec ainsi obtenu est repris par une solution d'acide chlorhydrique dans l'éthanol absolu. Le produit attendu précipite. Après essorage puis séchage, l'analyse du produit obtenu donne les résultats suivants :

| Analyse | Calculé pour $C_{12}H_{21}N_2O_4Cl_3$ | Trouvé |
|---|---|---|
| C% | 39,62 | 39,49 |
| H% | 5,78 | 5,67 |
| N% | 7,70 | 7,56 |
| O% | 17,61 | 17,81 |
| Cl% | 29,30 | 29,26 |

Exemple d'application

On prépare le mélange tinctorial suivant :

| | | |
|---|---|---|
| - Dichlorhydrate de 3,5-diamino-2,4-diméthoxy-chlorobenzène | | 0,68 g |
| - p-phénylènediamine | | 0,27 g |
| - Alcool oléique polyglycérolé à 2 moles de glycérol | | 4,5 g |
| - Alcool oléique polyglycérolé à 4 moles de glycérol | | 4,5 g |
| - ETHOMEEN O 12 - Société ARMOON HESS CHEMICAL Ltd (oléylamine oxyéthylénée à 12 moles d'O.E.) | | 4,5 g |
| - COMPERLAN KD - Société HENKEL (diéthanolamide de coprah) | | 9 g |
| - Propylène glycol | | 4 g |
| - 2-butoxyéthanol | | 8 g |
| - Ethanol à 96° | | 6 g |
| - MASQUOL DTPA - Société PROTEX | | 2 g |
| - Hydroquinone | | 0,15 g |
| - Solution de bisulfite de sodium à 35°Bé | | 1,3 g |
| - Ammoniaque à 22°Bé | | 10 g |
| - Eau | qsp | 100 g |
| - pH : 10 | | |

Au moment de l'emploi, on ajoute 100 g d'eau oxygénée à 20 volumes. Le mélange, appliqué 20 minutes à 35°C sur des cheveux décolorés leur confère après shampooing et rinçage une coloration brun gris foncé légèrement jaune.

Exemple d'application 2

On prépare le mélange tinctorial suivant :

| | |
|---|---|
| - Dichlorhydrate de 3,5-di(β-hydroxyéthyl)amino-2,4-diméthoxychlorobenzène | 0,91 g |
| - p-phénylènediamine | 0,27 g |
| - Alcool oléique polyglycérolé à 2 moles de glycérol | 4,5 g |
| - Alcool oléique polyglycérolé à 4 moles de glycérol | 4,5 g |
| - ETHOMEEN 0 12 - Société ARMOON HESS CHEMICAL Ltd (oléylamine oxyéthylénée à 12 moles d'O.E.) | 4,5 g |
| - COMPERLAN KD - Société HENKEL (diéthanolamide de coprah) | 9 g |
| - Propylène glycol | 4 g |
| - 2-butoxyéthanol | 8 g |
| - Ethanol à 96° | 6 g |
| - MASQUOL DTPA - Société PROTEX (sel pentasodique de l'acide diéthylène triamine pentacétique) | 2 g |
| - Hydroquinone | 0,15 g |
| - Solution de bisulfite de sodium à 35°Bé | 1,3 g |
| - Ammoniaque à 22°Bé | 10 g |
| - Eau qsp | 100 g |
| - pH : 10 | |

Au moment de l'emploi, on ajoute 100 g d'eau oxygénée à 20 volumes. Le mélange, appliqué 20 minutes à 35°C sur des cheveux décolorés, leur confère après shampooing et rinçage une coloration pourpre rouge foncé.

Exemple d'application 3

On prépare le mélange tinctorial suivant :

| | | |
|---|---|---|
| - Dichlorhydrate de 3-amino-5-méthylamino-2,4-diméthoxychlorobenzène | 0,723 | g |
| - p-phénylènediamine | 0,27 | g |
| - ALFOL C 16/18 - Société CONDEA (alcool cétylstéarylique) | 8 | g |
| - CIRE DE LANETTE E - Société HENKEL (sulfate cétylstéarylique de sodium) | 0,5 | g |
| - CEMULSOL B - RHONE POULENC (huile de ricin éthoxylée) | 1 | g |
| - Diéthanolamide oléique | 1,5 | g |
| - MASQUOL DTPA - Société PROTEX (sel pentasodique de l'acide diéthylène triamine pentacétique) | 2,5 | g |
| - Ammoniaque à 22°Bé | 11 | g |
| - Solution de bisulfite de sodium à 35°Bé | 1,3 | g |
| - Eau qsp | 100 | g |
| - pH : 9,7 | | |

Au moment de l'emploi, on ajoute 100 g d'eau oxygénée à 20 volumes. Le mélange, appliqué 20 minutes à 35° C sur des cheveux naturellement blancs à 90%, leur confère après shampooing et rinçage une coloration rouge pourpre foncé.

Exemple d'application 4

On prépare le mélange tinctorial suivant :

| | | |
|---|---|---|
| - Dichlorhydrate de 5-amino-3-(β-hydroxyéthyl)-amino-2,4-diméthoxychlorobenzène | 0,79 | g |
| - p-phénylènediamine | 0,27 | g |
| - Alcool oléique polyglycérolé de 2 moles de glycérol | 4,5 | g |
| - Alcool oléique polyglycérolé à 4 moles de glycérol | 4,5 | g |
| - ETHOMEEN O 12 - Société ARMOON HESS CHEMICAL Ltd - (oléylamine oxyéthylénée à 12 moles d'O.E.) | 4,5 | g |
| - COMPERLAN KD - Société HENKEL (diéthanolamide de coprah) | 9 | g |
| - Propylène glycol | 4 | g |
| - 2-butoxyéthanol | 8 | g |
| - Ethanol à 96° | 6 | g |
| - MASQUOL DTPA - Société PROTEX (sel pentasodique de l'acide diéthylène triamine pentacétique) | 2 | g |
| - Hydroquinone | 0,15 | g |
| - Solution de bisulfite de sodium à 35°Bé | 1,3 | g |
| - Ammoniaque à 22°Bé | 10 | g |
| - Eau qsp | 100 | g |
| - pH : 10 | | |

Au moment de l'emploi, on ajoute 100 g d'eau oxygénée à 20 volumes. Le mélange, appliqué 20 minutes à 30° C sur des cheveux décolorés, leur confère après shampooing et rinçage une coloration brun grisâtre.

Exemple d'application 5

On prépare le mélange tinctorial suivant :

| | |
|---|---|
| - Dichlorhydrate de 5-(β-hydroxyéthyl)amino-3-amino-2,4-diméthoxychlorobenzène | 0,79 g |
| - p-phénylènediamine | 0,27 g |
| - Alcool oléique polyglycérolé à 2 moles de glycérol | 4,5 g |
| - Alcool oléique polyglycérolé à 4 moles de glycérol | 4,5 g |
| - ETHOMEEN O 12 - Société ARMOON HESS CHEMICAL Ltd (oléylamine oxyéthylénée à 12 moles d'O.E.) | 9 g |
| - Propylène glycol | 4 g |
| - 2-butoxyéthanol | 8 g |
| - Ethanol à 96° | 6 g |
| - MASQUOL DTPA - Société PROTEX (sel pentasodique de l'acide diéthylène triamine pentacétique) | 2 g |
| - Hydroquinone | 0,15 g |
| - Solution de bisulfite de sodium à 35°Bé | 1,3 g |
| - Ammoniaque à 22°Bé | 10 g |
| - EAU qsp | 100 g |
| - pH : 10 | |

Au moment de l'emploi, on ajoute 100 g d'eau oxygénée à 20 volumes. Le mélange, appliqué 25 minutes à 35° C sur des cheveux gris naturels, leur confère après shampooing et rinçage une coloration rouge pourpre foncé.

Exemple d'application 6

On prépare le mélange tinctorial suivant :

| | | |
|---|---|---|
| - Dichlorhydrate de 5-(β-hydroxyéthyl)amino-3-amino-2,4-diméthoxychlorobenzène | | 1,11 g |
| - Dichlorhydrate de 4-amino-N-(β-méthoxyéthyl)-aniline | | 0,7 g |
| - CEMULSOL NP 4 - RHONE POULENC (nonyl phénol oxyéthyléné à 4 moles O.E.) | | 12 g |
| - CEMULSOL NP 9 - RHONE POULENC (nonyl phénol oxyéthyléné à 9 moles O.E.) | | 15 g |
| - Alcool oléique polyglycérolé à 2 moles de glycérol | | 1,5 g |
| - Alcool oléique polyglycérolé à 4 moles de glycérol | | 1,5 g |
| - Propylène glycol | | 6 g |
| - TRILON B (acide éthylène diamine tétracétique) | | 0,12 g |
| - Ammoniaque à 22°Bé | | 11 g |
| - Acide thioglycolique | | 0,6 g |
| - Eau | qsp | 100 g |
| - pH : 10,2 | | |

Au moment de l'emploi, on ajoute 100 g d'eau oxygénée à 20 volumes. Le mélange, appliqué 15 minutes à 35° C sur des cheveux décolorés, leur confère après shampooing et rinçage une coloration lilas foncé.

Exemple d'application 7

On prépare le mélange tinctorial suivant :

| | | |
|---|---|---|
| - Dichlorhydrate de 3,5-di(β-hydroxyéthyl)amino-2,4-diméthoxychlorobenzène | 1,09 | g |
| - 2,6-diméthylparaphénylènediamine | 0,63 | g |
| - ALFOL C 16/18 - Société CONDEA (alcool cétylstéarylique) | 19 | g |
| - EUTANOL G - Société HENKEL (2-octyldodécanol) | 4,5 | g |
| - MERGITAL C.S. - Société HENKEL (alcool cétyl stéarylique à 15 moles O.E.) | 2,5 | g |
| - Lauryl sulfate d'ammonium | 10 | g |
| - Polymère cationique présentant le motif récurrent suivant : 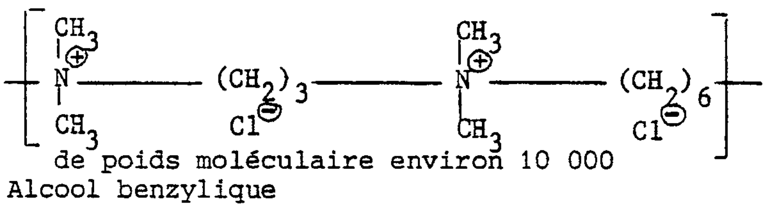 de poids moléculaire environ 10 000 | | |
| - Alcool benzylique | 2 | g |
| - Ammoniaque à 22°Bé | 11 | ml |
| - TRILON B (acide éthylènediamine tétracétique) | 1 | g |
| - Bisulfite de sodium à 35°Bé | 1,2 | g |
| - Eau qsp | 100 | g |
| - pH : 9,2 | | |

Au moment de l'emploi, on ajoute 100 g d'eau oxygénée à 20 volumes. Le mélange, appliqué 20 minutes à 35° C sur des cheveux décolorés, leur confère après shampooing et rinçage une coloration bleu pourpre.

Exemple d'application 8

On prépare le mélange tinctorial suivant :

| | |
|---|---|
| - Dichlorhydrate de 3,5-diamino-2,4-diméthoxychlorobenzène | 0,16 g |
| - Dichlorhydrate de 5-méthylamino-3-amino-2,4-diméthoxychlorobenzène | 0,20 g |
| - Dichlorhydrate de p-toluylènediamine | 0,58 g |
| - Métaaminophénol | 0,26 g |
| - Résorcine | 0,16 g |
| - CARBOPOL 934 - Société GOODRICH CHEMICALS | 3 g |
| - Alcool à 96° | 11 g |
| - 2-butoxyéthanol | 5 g |
| - Bromure de triméthyl cétyl ammonium | 2 g |
| - TRILON B (acide éthylène diamine tétracétique) | 0,2 g |
| - Ammoniaque à 22°Bé | 10 g |
| - Bisulfite de sodium à 35°Bé | 1 g |
| - Eau qsp | 100 g |
| - pH : 9,2 | |

Au moment de l'emploi, on ajoute 100 g d'eau oxygénée à 20 volumes. Le mélange, appliqué 20 minutes à 35° C sur des cheveux décolorés, leur confère après shampooing et rinçage une coloration nègre à reflets rouges.

**Revendications**

**Revendications pour les Etats contractants suivants : AT, BE, CH, DE, FR, GB, GR, IT, LI, NL, SE**

**1.** Composés répondant à la formule

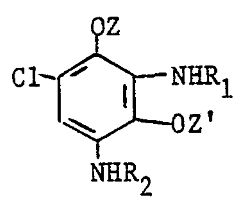 (I)

dans laquelle :

Z et Z′ représentent indépendamment l'un de l'autre un radical alkyle ayant de 1 à 4 atomes de carbone ou un radical hydroxyalkyle ayant de 2 à 4 atomes de carbone,

$R_1$ et $R_2$ représentent indépendamment l'un de l'autre un atome d'hydrogène, un radical alkyle ayant de 1 à 4 atomes de carbone ou mono- ou polyhydroxyalkyle ayant 2 ou 3 atomes de carbone, à condition que lorsque $R_1$ et $R_2$ désignent simultanément un atome d'hydrogène, Z et Z′ ne désignent pas simultanément un radical méthyle, et leurs sels d'addition avec un acide.

**2.** Composés selon la revendication 1, caractérisés par le fait qu'ils sont choisis parmi le 3-(β-hydroxyéthyl)amino-5-amino-2,4-diméthoxychlorobenzène, le 3-amino-5-(β-hydroxyéthyl)amino-2,4-diméthoxychlorobenzène, le 3,5-di(β-hydroxyéthyl)amino-2,4-diméthoxychlorobenzène,le 3-méthylamino-5-amino-2,4-diméthoxychlorobenzène, le 3,5-diamino-2,4-diéthoxychlorobenzène, le 3,5-diamino-2,4-di(γ-hydroxypropoxy)chlorobenzène et leurs sels d'addition avec un acide minéral.

**3.** Utilisation en tant que coupleur d'un composé de formule (I′)

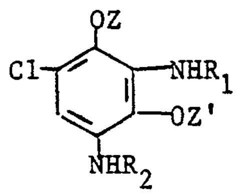

(I')

dans laquelle Z et Z' représentent indépendamment l'un de l'autre un radical alkyle ayant de 1 à 4 atomes de carbone ou hydroxyalkyle ayant de 2 à 4 atomes de carbone, $R_1$ et $R_2$ représentent indépendamment l'un de l'autre un atome d'hydrogène, un radical alkyle ayant de 1 à 4 atomes de carbone ou mono- ou polyhydroxyalkyle ayant 2 ou 3 atomes de carbone, ou d'un sel d'addition avec un acide de ce composé, pour la teinture d'oxydation des fibres kératiniques et en particulier des cheveux humains.

4. Composition de teinture capillaire, caractérisée par le fait qu'elle comprend, dans un support aqueux cosmétiquement acceptable, au moins un composé de formule (I') selon la revendication 3 ou l'un de ses sels d'addition avec un acide, à titre de coupleur, en association avec au moins un précurseur de colorant d'oxydation de type para.

5. Composition tinctoriale selon la revendication 4, caractérisée par le fait qu'elle contient, à titre de coupleur, un composé choisi parmi le 5-(β-hydroxyéthyl)amino-3-amino-2,4-diméthoxychlorobenzène, le 5-amino-3-(β-hydroxyéthyl)amino-2,4-diméthoxychloro benzène, le 3,5-di(β-hydroxyéthyl)amino-2,4-diméthoxychlorobenzène, le 5-méthylamino-3-amino-2,4-diméthoxychlorobenzène, le 3,5-diamino-2,4-diméthoxychlorobenzène, le 3,5-diamino-2,4-diéthoxychlorobenzène, le 3,5-diamino-2,4-di(γ-hydroxypropoxy)chlorobenzène ou leurs sels d'addition avec un acide minéral.

6. Composition tinctoriale selon la revendication 4 ou 5, caractérisée par le fait qu'elle contient 0,05 à 3,5% en poids de composé (I') ou de l'un de ses sels d'addition avec un acide, sur la base du poids total de la composition.

7. Composition tinctoriale selon l'une quelconque des revendications 4 à 6, caractérisée par le fait que le précurseur de colorant d'oxydation de type para est choisi parmi les paraphénylène diamines, les para-aminophénols, les composés para-hétérocycliques ou leurs mélanges.

8. Composition tinctoriale selon la revendication 7, caractérisée par le fait que les paraphénylènediamines répondent à la formule :

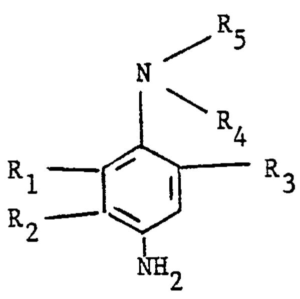

(VIII)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représente un atome d'hydrogène lorsque $R_4$ et $R_5$ ne représentent pas un atome d'hydrogène, ou sont des sels de composés de formule (VIII).

**9.** Composition tinctoriale selon la revendication 8, caractérisée par le fait qu'elle ccntient au moins une paraphénylènediamine choisie parmi la p-phénylènediamine, l'isopropyl p-phénylènediamine, la p-toluylènediamine, la méthoxyparaphénylènediamine, la chloroparaphénylènediamine, la 2,6-diméthyl-para-phénylènediamine, la 2,3-diméthyl-paraphénylènediamine, la 2,5-diméthylparaphénylènediamine, la 2-méthyl-5-méthoxyparaphénylènediamine, la 2,6-diméthyl-5-méthoxyparaphénylènediamine, la N,N-diméthylparaphénylènediamine, la 3-méthyl-4-amino-N,N-diéthylaniline, la N,N-di(β-hydroxyéthyl) paraphénylènediamine, la 3-méthyl-4-amino-N,N-di(β-hydroxyéthyl) aniline, la 3-chloro-4-amino-N,N-di-(β-hydroxyéthyl)aniline, la 4-amino-N,N-(éthyl, carbamylméthyl)aniline, la 3-méthyl-4-amino N,N-(éthyl, carbamylméthyl)aniline, la 4-amino-N,N-(éthyl, β-pipéridinoéthyl)aniline, la 3-méthyl-4-amino-N,N-(éthyl, β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)aniline, la 4-amino-N, β-méthoxyéthylaniline, la 3-méthyl-4-amino-N,N-(éthyl, β-acétylaminoéthyl) aniline, la 4-amino-N,N-(éthyl, β-mésylaminoéthyl)aniline, la 3-méthyl-4-amino-N,N-(éthyl, β-mésylaminoéthyl)aniline, la 4-amino- N,N-(éthyl, β-sulfoéthyl)aniline, la 3-méthyl-4-amino-N,N-(éthyl, β-sulfoéthyl)aniline, la N-[(4′-amino)phényl]-morpholine, la N-[(4′-amino)phényl] pipéridine, sous forme de base libre ou sous forme de sel cosmétiquement acceptable.

**10.** Composition tinctoriale selon la revendication 9, caractérisée par le fait qu'elle contient au moins un composé de formule (I′) dans laquelle $R_2$ est différent d'un atome d'hydrogène ou l'un de ses sels avec un acide, à titre de coupleur, en association avec au moins une paraphénylènediamine telle que définie dans la revendication 9.

**11.** Composition tinctoriale selon la revendication 9, caractérisée par le fait qu'elle contient au moins un composé de formule (I′) dans laquelle $R_2$ est un atome d'hydrogène ou l'un de ses sels avec un acide, à titre de coupleur, en association avec au moins une paraphénylènediamine telle que définie dans la revendication 9.

**12.** Composition tinctoriale selon la revendication 7, caractérisée par le fait qu'elle contient au moins un para-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

**13.** Composition tinctoriale selon la revendication 7, caractérisée par le fait que le précurseur de colorant d'oxydation de type para est un composé para hétérocyclique choisi parmi la 2,5-diaminopyridine, la 2-hydroxy-5-aminopyridine et la tétraaminopyrimidine.

**14.** Composition tinctoriale selon l'une quelconque des revendications 4 à 13, caractérisée par le fait qu'elle contient d'autres coupleurs choisis parmi les métadiphénols, les métaaminophénols, les métaphénylènediamines, les métaacylaminophénols, les métauréidophénols, les métacarbalcoxyaminophénols, 1′∝-naphtol, les composés β-cétoniques et les pyrazolones.

**15.** Composition tinctoriale selon l'une quelconque des revendications 4 à 14, caractérisée par le fait que sa concentration totale en précurseurs de colorants d'oxydation de type para et coupleurs est comprise entre 0,1 et 7% en poids.

**16.** Composition tinctoriale selon l'une quelconque des revendications 4 à 15, caractérisée par le fait qu'elle contient en outre des précurseurs de colorants de type ortho choisis parmi les orthoaminophénols, les orthophénylènediamines et les orthodiphénols.

**17.** Composition tinctoriale selon l'une quelconque des revendications 4 à 16, caractérisée par le fait qu'elle contient en outre des colorants directs choisis parmi les colorants azoïques, anthraquinoniques et les dérivés nitrés de la série benzénique.

**18.** Composition tinctoriale selon l'une quelconque des revendications 4 à 17, caractérisée par le fait qu'elle a un pH compris entre 8 et 11, et de préférence compris entre 9 et 11.

**19.** Composition tinctoriale selon l'une quelconque des revendications 4 à 18, caractérisée par le fait qu'elle contient 1 à 40% en poids d'un solvant organique choisi parmi les alcanols inférieurs, le glycérol, les glycols ou éthers de glycols, et leurs mélanges.

**20.** Composition tinctoriale selon l'une quelconque des revendications 4 à 19, caractérisée par le fait qu'elle contient en outre 0,5 à 40% en poids d'au moins un agent tensio-actif anionique, cationique, non ionique, amphotère ou leurs mélanges.

**21.** Composition tinctoriale selon l'une quelconque des revendications 4 à 20, caractérisée par le fait qu'elle contient en outre des adjuvants cosmétiques choisis parmi les épaississants, les agents anti-oxydants, les agents de pénétration, les agents séquestrants, les tampons, les parfums, les agents alcalinisants, les propulseurs.

**22.** Procédé de teinture capillaire suivant un procédé mettant en oeuvre la révélation par un oxydant, caractérisé par le fait qu'il consiste à mélanger au moment de l'emploi la composition tinctoriale selon l'une quelconque des revendications 4 à 21 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à nouveau et à les sécher.

**23.** Procédé de teinture capillaire suivant un procédé mettant en oeuvre la révélation par un oxydant, caractérisé par le fait qu'il consiste à appliquer dans un premier temps sur les cheveux une composition tinctoriale contenant au moins un précurseur de colorant d'oxydation de type para tel que défini dans l'une des revendications 7 à 13 puis à appliquer dans un second temps une composition tinctoriale contenant un composé de formule (I') ou l'un de ses sels, l'agent oxydant étant présent dans la composition appliquée dans le second temps ou bien appliqué sur les cheveux eux-mêmes dans un troisième temps, à laisser poser pendant 10 à 40 minutes, de préférence 15 à 30 minutes, puis à rincer les cheveux, à les laver au shampooing, à les rincer à nouveau et à les sécher.

**Revendications pour l'Etat contractant suivant: ES**

**1.** Utilisation en tant que coupleur d'un composé de formule (I')

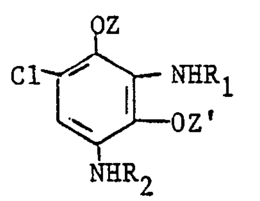

(I')

dans laquelle Z et Z' représentent indépendamment l'un de l'autre un radical alkyle ayant de 1 à 4 atomes de carbone ou hydroxyalkyle ayant de 2 à 4 atomes de carbone, $R_1$ et $R_2$ représentent indépendamment l'un de l'autre un atome d'hydrogène, un radical alkyle ayant de 1 à 4 atomes de carbone ou mono- ou polyhydroxyalkyle ayant 2 ou 3 atomes de carbone, ou d'un sel d'addition avec un acide de ce composé, pour la teinture d'oxydation des fibres kératiniques et en particulier des cheveux humains.

**2.** Composition de teinture capillaire, caractérisée par le fait qu'elle comprend, dans un support aqueux cosmétiquement acceptable, au moins un composé de formule (I') selon la revendication 1 ou l'un de ses sels d'addition avec un acide, à titre de coupleur, en association avec au moins un précurseur de colorant d'oxydation de type para.

**3.** Composition tinctoriale selon la revendication 2, caractérisée par le fait qu'elle contient, à titre de coupleur, un composé choisi parmi le 5-($\beta$-hydroxyéthyl)amino-3-amino-2,4-diméthoxychlorobenzène, le 5-amino-3-($\beta$-hydroxyéthyl)amino-2,4-diméthoxychlorobenzène, le 3,5-di($\beta$-hydroxyéthyl)amino-2,4-diméthoxychloro benzène, le 5-méthylamino-3-amino-2,4-diméthoxychlorobenzène, le 3,5-diamino-2,4-diméthoxychlorobenzène, le 3,5-diamino-2,4-diéthoxychlorobenzène, le 3,5-diamino-2,4-di($\gamma$-hydroxy-

propoxy)chlorobenzène ou leurs sels d'addition avec un acide minéral.

4. Composition tinctoriale selon la revendication 2 ou 3, caractérisée par le fait qu'elle contient 0,05 à 3,5% en poids de composé (I') ou de l'un de ses sels d'addition avec un acide, sur la base du poids total de la composition.

5. Composition tinctoriale selon l'une quelconque des revendications 2 à 4, caractérisée par le fait que le précurseur de colorant d'oxydation de type para est choisi parmi les paraphénylène diamines, les para-aminophénols, les composés para-hétérocyliques ou leurs mélanges.

6. Composition tinctoriale selon la revendication 5, caractérisée par le fait que les paraphénylènediamines répondent à la formule :

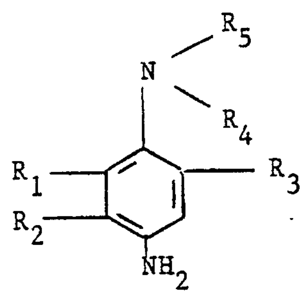

(VIII)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ et $R_3$ représente un atome d'hydrogène lorsque $R_4$ et $R_5$ ne représentent pas un atome d'hydrogène, ou sont des sels de composés de formule (VIII).

7. Composition tinctoriale selon la revendication 6, caractérisée par le fait qu'elle contient au moins une paraphénylènediamine choisie parmi la p-phénylènediamine, l'isopropyl p-phénylènediamine, la p-toluylènediamine, la méthoxyparaphénylènediamine, la chloroparaphénylènediamine, la 2,6-diméthyl-para-phénylènediamine, la 2,3-diméthyl-paraphénylènediamine, la 2,5-diméthylparaphénylènediamine, la 2-méthyl-5-méthoxyparaphénylènediamine, la 2,6-diméthyl-5-méthoxyparaphénylènediamine, la N,N-diméthylparaphénylènediamine, la 3-méthyl-4-amino-N,N-diéthylaniline, la N,N-di($\beta$-hydroxyéthyl) paraphénylènediamine, la 3-méthyl-4-amino-N,N-di($\beta$-hydroxyéthyl)aniline, la 3-chloro-4-amino-N,N-di-($\beta$-hydroxyéthyl)aniline, la 4-amino-N,N-(éthyl, carbamylméthyl)aniline, la 3-méthyl-4-amino N,N-(éthyl, carbamylméthyl)aniline, la 4-amino-N,N-(éthyl, $\beta$-pipéridinoéthyl)aniline, la 3-méthyl-4-amino-N,N-(éthyl, $\beta$-pipéridinoéthyl)aniline, la 4-amino-N,N-(éthyl, $\beta$-morpholinoéthyl)aniline, la 3-méthyl-4-amino-N,N-(éthyl, $\beta$-morpholinoéthyl)aniline, la 4-amino-N,N-(éthyl, $\beta$-acétylaminoéthyl)aniline, la 4-amino-N, -méthoxyéthylaniline, la 3-méthyl-4-amino-N,N-(éthyl, $\beta$-acétylaminoéthyl) aniline, la 4-amino-N,N-(éthyl, $\beta$-mésylaminoéthyl)aniline, la 3-méthyl-4-amino-N,N-(éthyl, $\beta$-mésylaminoéthyl)aniline, la 4-amino-N,N-(éthyl, $\beta$-sulfoéthyl)aniline, la 3-méthyl-4-amino-N,N-(éthyl, $\beta$-sulfoéthyl)aniline, la N-[(4'-amino)phényl]-morpholine, la N-[(4'-amino)phényl] pipéridine, sous forme de base libre ou sous forme de sel cosmétiquement acceptable.

8. Composition tinctoriale selon la revendication 2, caractérisée par le fait qu'elle contient au moins un composé de formule (I') dans laquelle $R_2$ est différent d'un atome d'hydrogène ou l'un de ses sels avec un acide, à titre de coupleur, en association avec au moins une paraphénylènediamine telle que définie dans la revendication 7.

9. Composition tinctoriale selon la revendication 2, caractérisée par le fait qu'elle contient au moins un composé de formule (I') dans laquelle $R_2$ est un atome d'hydrogène ou l'un de ses sels avec un acide,

à titre de coupleur, en association avec au moins une paraphénylènediamine telle que définie dans la revendication 7.

**10.** Composition tinctoriale selon la revendication 5, caractérisée par le fait qu'elle contient au moins un para-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-($\beta$-hydroxyéthyl)-4-aminophénol, le 2-méthoxy-4-aminophénol, le 3-méthoxy-4-aminophénol.

**11.** Composition tinctoriale selon la revendication 5, caractérisée par le fait que le précurseur de colorant d'oxydation de type para est un composé para hétérocyclique choisi parmi la 2,5-diaminopyridine, la 2-hydroxy-5-aminopyridine et la tétraaminopyrimidine.

**12.** Composition tinctoriale selon l'une quelconque des revendications 2 à 11, caractérisée par le fait qu'elle contient d'autres coupleurs choisis parmi les métadiphénols, les métaaminophénols, les métaphénylènediamines, les métaacylaminophénols, les métauréidophénols, les métacarbalcoxyaminophénols, l'$\alpha$-naphtol, les composés $\beta$-cétoniques et les pyrazolones.

**13.** Composition tinctoriale selon l'une quelconque des revendications 2 à 12, caractérisée par le fait que se concentration totale en précurseurs de colorants d'oxydation de type para et coupleurs est comprise entre 0,1 et 7% en poids.

**14.** Composition tinctoriale selon l'une quelconque des revendications 2 à 13, caractérisée par le fait qu'elle contient en outre des précurseurs de colorants de type ortho choisis parmi les orthoaminophénols, les orthophénylènediamines et les orthodiphénols.

**15.** Composition tinctoriale selon l'une quelconque des revendications 2 à 14, caractérisée par le fait qu'elle contient en outre des colorants directs choisis parmi les colorants azoïques, anthraquinoniques et les dérivés nitrés de la série benzénique.

**16.** Composition tinctoriale selon l'une quelconque des revendications 2 à 15, caractérisée par le fait qu'elle a un pH compris entre 8 et 11, et de préférence compris entre 9 et 11.

**17.** Composition tinctoriale selon l'une quelconque des revendications 2 à 16, caractérisée par le fait qu'elle contient 1 à 40% en poids d'un solvant organique choisi parmi les alcanols inférieurs, le glycérol, les glycols ou éthers de glycols, et leurs mélanges.

**18.** Composition tinctoriale selon l'une quelconque des revendications 2 à 17, caractérisée par le fait qu'elle contient en outre 0,5 à 40% en poids d'au moins un agent tensio-actif anionique, cationique, non ionique, amphotère ou leurs mélanges.

**19.** Composition tinctoriale selon l'une quelconque des revendications 2 à 18, caractérisée par le fait qu'elle contient en outre des adjuvants cosmétiques choisis parmi les épaississants, les agents anti-oxydants, les agents de pénétration, les agents séquestrants, les tampons, les parfums, les agents alcalinisants, les propulseurs.

**20.** Procédé de teinture capillaire suivant un procédé mettant en oeuvre la révélation par un oxydant, caractérisé par le fait qu'il consiste à mélanger au moment de l'emploi la composition tinctoriale selon l'une quelconque des revendications 2 à 19 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à nouveau et à les sécher.

**21.** Procédé de teinture capillaire suivant un procédé mettant en oeuvre la révélation par un oxydant, caractérisé par le fait qu'il consiste à appliquer dans un premier temps sur les cheveux une composition tinctoriale contenant au moins un précurseur de colorant d'oxydation de type para tel que défini dans l'une des revendications 5 à 11 puis à appliquer dans un second temps une composition

tinctoriale contenant un composé de formule (I′) ou l'un de ses sels, l'agent oxydant étant présent dans la composition appliquée dans le second temps ou bien appliqué sur les cheveux eux-mêmes dans un troisième temps, à laisser poser pendant 10 à 40 minutes, de préférence 15 à 30 minutes, puis à rincer les cheveux, à les laver au shampooing, à les rincer à nouveau et à les sécher.

**22.** Procédé de préparation d'une composition de teinture capillaire, caractérisé par le fait qu'il consiste à dissoudre dans un support aqueux cosmétiquement acceptable, à titre de coupleur, 0,05 à 3,5% en poids d'au moins un composé de formule (I′)

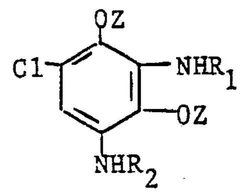

(I')

dans laquelle Z et Z′ représentent indépendamment l'un de l'autre un radical alkyle ayant de 1 à 4 atomes de carbone ou hydroxyalkyle ayant de 2 à 4 atomes de carbone, $R_1$ et $R_2$ représentent indépendamment l'un de l'autre un radical alkyle ayant de 1 à 4 atomes de carbone ou mono-ou polyhydroxyalkyle ayant 2 ou 3 atomes de carbone, ou d'un sel d'addition avec un acide de ce composé, cette quantité étant calculée sur la base du poids total de la composition, et au moins un précurseur de colorant d'oxydation de type para, la concentration totale en coupleurs et en précurseurs de colorants d'oxydation de type para étant comprise entre 0,1 et 7% en poids sur la base du poids total de la composition, puis à ajouter au moins un adjuvant cosmétique choisi parmi les tensio-actifs anioniques, cationiques, non-ioniques, amphotères ou leurs mélanges, les solvants organiques, les épaississants, les agents anti-oxydants, les agents de pénétration, les agents séquestrants, les tampons et les parfums, et enfin à adjuster le pH de la composition de teinture à une valeur comprise entre 8 et 11, et de préférence entre 9 et 11, à l'aide d'un agent alcalinisant.

**23.** Procédé selon la revendication 22, caractérisé par le fait qu'on utilise à titre de coupleur, au moins un composé choisi parmi le 5-(β-hydroxyéthyl)amino-3-amino-2,4-diméthoxychlorobenzène, le 5-amino-3-(β-hydroxyéthyl)amino-2,4-diméthoxychloro benzène, le 3,5-di(β-hydroxyéthyl)amino-2,4-diméthoxychlorobenzène, le 5-méthylamino-3-amino-2,4-diméthoxychlorobenzène, le 3,5-diamino-2,4-diméthoxychlorobenzène, le 3,5-diamino-2,4-diéthoxychlorobenzène, le 3,5-diamino-2,4-di(γ-hydroxypropoxy)-chlorobenzène ou leurs sels d'addition avec un acide minéral.

**24.** Procédé selon la revendication 22 ou 23, caractérisé par le fait qu'on utilise un précuseur de colorant d'oxydation de type para choisi parmi les paraphénylène diamines, les para-aminophénols, les composés para-hétérocycliques ou leurs mélanges.

**25.** Procédé selon la revendication 24, caractérisé par le fait qu'on utilise une paraphénylène diamine répondant à la formule :

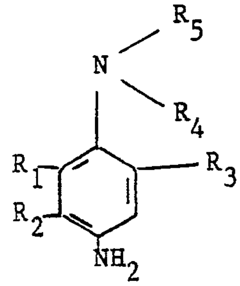

(VIII)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle,

carbétoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou un sel d'un composé de formule (VIII).

**26.** Procédé selon la revendication 25, caractérisé par le fait qu'on utilise une paraphénylène diamine choisie parmi la p-phénylènediamine, l'isopropyl p-phénylènediamine, la p-toluylènediamine, la méthoxyparaphénylène diamine, la chloroparaphénylènediamine, la 2,6-diméthyl-para-phénylènediamine, la 2,3-diméthyl-paraphénylènediamine, la 2,5-diméthylparaphénylènediamine, la 2-méthyl-5-méthoxyparaphénylènediamine, la 2,6-diméthyl-5-méthoxyparaphénylènediamine, la N,N-diméthylparaphénylènediamine, la 3-méthyl-4-amino-N,N-diéthylaniline, la N,N-di($\beta$-hydroxyéthyl) paraphénylènediamine, la 3-méthyl-4-amino-N,N-di($\beta$-hydroxyéthyl) aniline, la 3-chloro-4-amino-N,N-di-($\beta$-hydroxyéthyl)aniline, la 4-amino-N,N-(éthyl, carbamylméthyl)aniline, la 3-méthyl-4-amino N,N-(éthyl, carbamylméthyl)aniline, la 4-amino-N,N-(éthyl, $\beta$-pipéridinoéthyl)aniline, la 3-méthyl-4-amino-N,N-(éthyl, $\beta$-pipéridinoéthyl)aniline, la 4-amino-N,N-(éthyl, $\beta$-morpholinoéthyl)aniline, la 3-méthyl-4-amino-N,N-(éthyl, $\beta$-morpholinoéthyl)-aniline, la 4-amino-N,N-(éthyl, $\beta$-acétylaminoéthyl)aniline, la 4-amino-N, $\beta$-méthoxyéthylaniline, la 3-méthyl-4-amino-N,N-(éthyl, $\beta$-acétylaminoéthyl) aniline, la 4-amino-N,N-(éthyl, $\beta$-mésylaminoéthyl)-aniline, la 3-méthyl-4-amino-N,N-(éthyl, $\beta$-mésylaminoéthyl)aniline, la 4-amino- N,N-(éthyl, $\beta$-sulfoéthyl)-aniline, la 3-méthyl-4-amino-N,N-(éthyl, $\beta$-sulfoéthyl)aniline, la N-[(4′-amino)phényl]morpholine, la N-[-(4′-amino)phényl] pipéridine, sous forme de base libre ou sous forme de sel cosmétiquement acceptable.

**27.** Procédé selon la revendication 22, caractérisé par le fait qu'on utilise un composé de formule (I′) dans laquelle $R_2$ est différent d'un atome d'hydrogène ou l'un de ses sels avec un acide, à titre de coupleur, en association avec au moins une paraphénylènediamine telle que définie dans la revendication 26.

**28.** Procédé selon la revendication 22, caractérisé par le fait qu'on utilise un composé de formule (I′) dans laquelle $R_2$ est un atome d'hydrogène ou l'un de ses sels avec un acide, à titre de coupleur, en association avec au moins une paraphénylènediamine telle que définie dans la revendication 26.

**29.** Procédé selon la revendication 24, caractérisé par le fait qu'on utilise un para-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-($\beta$-hydroxyéthyl)-4-aminophénol, le 2-méthoxy-4-aminophénol, le 3-méthoxy-4-aminophénol.

**30.** Procédé selon la revendication 24, caractérisé par le fait qu'on utilise à titre de précurseur de colorants d'oxydation de type para un composé para hétérocyclique choisi parmi la 2,5-diaminopyridine, la 2-hydroxy-5-aminopyridine et la tétraminopyrimidine.

**31.** Procédé selon l'une quelconque des revendications 22 à 30, caractérisé par le fait qu'on ajoute d'autres coupleurs choisis par les métadiphénols, les métaaminophénols, les métaphénylènediamines, les métaacylaminophénols, les métauréidophénols, les métacarbalcoxyaminophénols, l'$\alpha$-naphtol, les composés $\beta$-cétoniques et les pyrazolones.

**32.** Procédé selon l'une quelconque des revendications 22 à 31, caractérisé par le fait que l'on ajoute également des précurseurs de colorants de type ortho choisis parmi les orthoaminophénols, les orthophénylènediamines et les orthodiphénols.

**33.** Procédé selon l'une quelconque des revendications 22 à 32, caractérisé par le fait qu'on ajoute 1 à 40% en poids d'un solvant organique choisi parmi les alcanols inférieurs, le glycérol, les glycols ou éthers de glycols, et leurs mélanges.

**34.** Procédé selon l'une quelconque des revendications 22 à 33, caractérisé par le fait qu'on ajoute 0,5 à 40% en poids d'au moins un agent tensio-actif anionique, cationique, non ionique, amphotère ou leurs mélanges.

## Claims

**Claims for the following Contracting States : DE, GB, FR, AT, SE, IT, NL, CH, BE, GR, LI**

1. Compounds corresponding to the formula

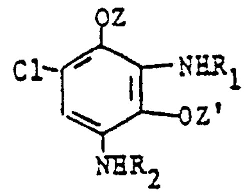

(I)

in which:

Z and Z' independently of one another represent an alkyl radical having from 1 to 4 carbon atoms or a hydroxyalkyl radical having from 2 to 4 carbon atoms, and

$R_1$ and $R_2$ independently of one another represent a hydrogen atom, an alkyl radical having from 1 to 4 carbon atoms or a mono- or polyhydroxyalkyl radical having 2 or 3 carbon atoms, on condition that when $R_1$ and $R_2$ simultaneously denote a hydrogen atom, Z and Z' do not simultaneously denote a methyl radical, and their addition salts with an acid.

2. Compounds according to Claim 1, characterised in that they are chosen from 3-(β-hydroxyethyl)amino-5-amino-2 , 4-dimethoxychlorobenzene, 3-amino-5-(β-hydroxyethyl)amino-2,4-dimethoxychlorobenzene, 3,5-di(β-hydroxyethyl)amino-2,4-dimethoxychlorobenzene, 3-methylamino-5-amino-2,4-dimethoxychlorobenzene, 3,5-diamino-2,4-diethoxychlorobenzene, 3,5-diamino-2,4-di(γ-hydroxypropoxy)-chlorobenzene and their addition salts with an inorganic acid.

3. Use of a compound of formula (I')

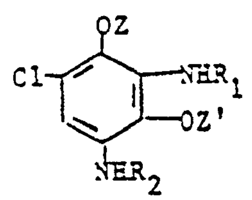

(I')

in which

Z and Z' independently of one another represent an alkyl radical having from 1 to 4 carbon atoms or a hydroxyalkyl radical having from 2 to 4 carbon atoms, and

$R_1$ and $R_2$ independently of one another represent a hydrogen atom, an alkyl radical having from 1 to 4 carbon atoms or a mono- or polyhydroxyalkyl radical having 2 or 3 carbon atoms, or an addition salt of this compound with an acid, as a coupler for the oxidation dyeing of keratinous fibres and in particular human hair.

4. A composition for dyeing hair, characterised in that it comprises, in an aqueous, cosmetically acceptable carrier, at least one compound of formula (I') according to Claim 3 or one of its addition salts with an acid, as coupler, in combination with at least one para-type oxidation dye precursor.

5. The dyeing composition according to Claim 4, characterised in that it contains, as a coupler, a compound chosen from 5-(β-hydroxyethyl)amino-3-amino-2,4-dimethoxychlorobenzene, 5-amino-3-(β-hydroxyethyl)amino-2,4-dimethoxychlorobenzene,3,5-di(β-hydroxyethyl)amino-2,4-dimethoxychlorobenzene, 5-methylamino-3-amino-2,4-dimethoxychlorobenzene, 3,5-diamino-2,4-dimethoxychlorobenzene, 3,5-diamino-2,4-diethoxychlorobenzene, 3,5-diamino-2,4-di(γ-hydroxypropoxy)chlorobenzene or their addition salts with an inorganic acid.

6. The dyeing composition according to Claim 4 or 5, characterised in that it contains 0.05 to 3.5% by weight of compound (I') or of one of its addition salts with an acid, based on the total weight of the

composition.

7. The dyeing composition according to any one of Claims 4 to 6, characterised in that the para-type oxidation dye precursor is chosen from para-phenylenediamines; para-aminophenols, para-heterocyclic compounds or their mixtures.

8. The dyeing composition according to Claim 7, characterised in that the para-phenylenediamines correspond to the formula:

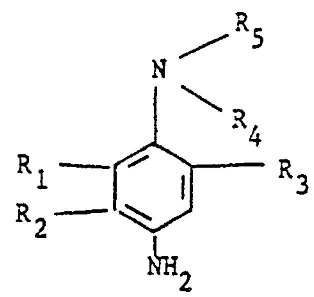

(VIII)

in which
$R_1$, $R_2$ and $R_3$ are identical or different and represent a hydrogen or halogen atom, an alkyl radical having 1 to 4 carbon atoms or an alkoxy radical having 1 to 4 carbon atoms, and
$R_4$ and $R_5$ are identical or different and represent a hydrogen atom, an alkyl, hydroxyalkyl, alkoxyalkyl, carbamylalkyl, mesylaminoalkyl, acetylaminoalkyl, ureidoalkyl, oarbethoxyaminoalkyl, piperidinoalkyl or morpholinoalkyl radical, the alkyl or alkoxy groups representd by $R_4$ and $R_5$ having from 1 to 4 carbon atoms, or
$R_4$ and $R_5$, together with the nitrogen atom to which they are bonded, form a piperidino or morpholino heterocycle with the proviso that $R_1$ or $R_3$ represents a hydrogen atom when $R_4$ and $R_5$ do not represent a hydrogen atom, or are the salts of compounds of formula (VIII).

9. The dyeing composition according to Claim 8, characterised in that it contains at least one para-phenylenediamine chosen from p-phenylenediamine, isopropyl-p-phenylenediamine, p-toluylenediamine, methoxy-para-phenylenediamine, chloro-para-phenylenediamine, 2,6-dimethyl-para-phenylenediamine, 2,3-dimethyl-para-phenylenediamine, 2,5-dimethyl-para-phenylenediamine, 2-methyl-5-methoxy-para-phenylenediamine, 2, 6-dimethyl-5-methoxy-para-phenylenediamine, N,N-dimethyl-para-phenylenediamine, 3-methyl-4-amino-N,N-diethylaniline, N,N-di($\beta$-hydroxyethyl)-para-phenylenediamine, 3-methyl-4-amino-N,N-di($\beta$-hydroxyethyl)aniline, 3-chloro-4-amino-N,N-di-($\beta$-hydroxyethyl)aniline, 4-amino-N-ethyl-N-(carbamylmethyl)aniline, 3-methyl-4-amino-N-ethyl-N-(carbamylmethyl)aniline, 4-amino-N-ethyl-N-($\beta$-piperidinoethyl)aniline, 3-methyl-4-amino-N-ethyl-N-($\beta$-piperidinoethyl)aniline, 4-amino-N-ethyl-N-($\beta$-morpholinoethyl)aniline, 3-methyl-4-amino-N-ethyl-N-($\beta$-morpholinoethyl)aniline, 4-amino-N-ethyl-N-($\beta$-acetylaminoethyl)aniline, 4-amino-N-($\beta$-methoxyethyl)-aniline, 3-methyl-4-amino-N-ethyl-N-($\beta$-acetylaminoethyl)aniline, 4-amino-N-ethyl-N-($\beta$-mesylaminoethyl)aniline, 3-methyl-4-amino-N-ethyl-N-($\beta$-mesylaminoethyl)aniline, 4-amino-N-ethyl-N-($\beta$-sulphoethyl)aniline, 3-methyl-4-amino-N-ethyl-N-($\beta$-sulphoethyl)aniline, N-[(4'-amino)phenyl]morpholine and N-[(4'-amino)phenyl]piperidinein the form of a free base or in the form of a cosmetically acceptable salt.

10. The dyeing composition according to Claim 9, characterised in that it contains at least one compound of formula (I') in which $R_2$ is other than a hydrogen atom, or one of its salts with an acid, as a coupler ,in combination with at least one para-phenylenediamine as defined in Claim 9.

11. The dyeing composition according to Claim 9, characterised in that it contains at least one compound of formula (I') in which $R_2$ is a hydrogen atom, or one of its salts with an acid, as a coupler, in combination with at least one para-phenylenediamine as defined in Claim 9.

12. The dyeing composition according to Claim 7, characterised in that it contains at least one para-aminophenol chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

**13.** The dyeing composition according to Claim 7, characterised in that the para-type oxidation dye precursor is a para heterocyclic compound chosen from 2,5-diaminopyridine, 2-hydroxy-5-aminopyridine and tetraaminopyrimidine.

**14.** The dyeing composition according to any one of Claims 4 to 13, characterised in that it contains other couplers chosen from meta-diphenols, meta-aminophenols, meta-phenylenediamines, meta-acylaminophenols, meta-ureidophenols, meta-carbalkoxyaminophenols, α-naphthol, β-ketone compounds and pyrazolones.

**15.** The dyeing composition according to any one of Claims 4 to 14, characterised in that its total concentration of para-type oxidation dye precursors and couplers is between 0.1 and 7% by weight.

**16.** The dyeing composition according to any one of Claims 4 to 15, characterised in that it also contains ortho-type dye precursors chosen from ortho-aminophenols, ortho-phenylenediamines and ortho-diphenols.

**17.** The dyeing composition according to any one of Claims 4 to 16, characterised in that it also contains direct dyes chosen from azo and anthraquinone dyes and the nitro derivatives of the benzene series.

**18.** The dyeing composition according to any one of Claims 4 to 17, characterised in that it has a pH of between 8 and 11 and preferably of between 9 and 11.

**19.** The dyeing composition according to any one of Claims 4 to 18, characterised in that it contains 1 to 40% by weight of an organic solvent chosen from lower alkanols, glycerol, glycols or glycol ethers, and their mixtures.

**20.** The dyeing composition according to any one of Claims 4 to 19, characterised in that it also contains 0.5 to 40% by weight of at least one anionic, cationic, nonionic or amphoteric surfactant or their mixtures.

**21.** The dyeing composition according to any one of Claims 4 to 20, characterised in that it also contains cosmetic adjuvants chosen from thickeners, antioxidants, penetration agents, sequestering agents, buffers, perfumes, alkalinising agents and propellants.

**22.** A method for dyeing hair in accordance with a method using development be an oxidant, characterised in that it consists in mixing the dyeing composition according to any one of Claims 4 to 21, at the time of use, with an oxidising solution in a sufficient amount, then applying the mixture obtained to the hair, leaving said mixture on the hair for 10 to 40 minutes, preferably 15 to 30 minutes, then rinsing the hair, washing it with shampoo, rinsing it again and drying it.

**23.** A method for dyeing hair in accordance with a method using development by an oxidant, characterised in that it consists in applying to the hair, in a first step, a dyeing composition containing at least one para-type oxidation dye precursor as defined in one of Claims 7 to 13, then, in a second step, applying a dyeing composition containing a compound of formula (I') or one of its salts, the oxidising agent being present in the composition applied in the second step or being applied to the hair itself in a third step, leaving the composition on the hair for 10 to 40 minutes, preferably 15 to 30 minutes, then rinsing the hair, washing it with shampoo, rinsing it again and drying it.

**Claims for the following Contracting State : ES**

**1.** Use of a compound of formula (I')

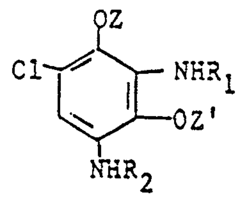

(I')

in which
Z and Z' independently of one another represent an alkyl radical having from 1 to 4 carbon atoms or a hydroxyalkyl radical having from 2 to 4 carbon atoms, and
$R_1$ and $R_2$ independently of one another represent a hydrogen atom, an alkyl radical having from 1 to 4 carbon atoms or a mono- or polyhydroxyalkyl radical having 2 or 3 carbon atoms, or an addition salt of this compound with an acid, as a coupler for the oxidation dyeing of keratinous fibres and in particular human hair.

**2.** Composition for dyeing hair, characterised in that it comprises, in an aqueous, cosmetically acceptable carrier, at least one compound of formula (I') according to Claim 1 or one of its addition salts with an acid, as coupler, in combination with at least one para-type oxidation dye precursor.

**3.** The dyeing composition according to Claim 2, characterised in that it contains, as coupler, a compound chosen from 5-(β-hydroxyethyl)amino-3-amino-2,4-dimethoxychlorobenzene, 5-amino-3-(β-hydroxyethyl)amino-2,4-dimethoxychlorobenzene,3,5-di(β-hydroxyethyl)amino-2,4-dimethoxychlorobenzene, 5-methylamino-3-amino-2,4-dimethoxychlorobenzene, 3,5-diamino-2,4-dimethoxychlorobenzene, 3,5-diamino-2,4-diethoxychlorobenzene, 3,5-diamino-2,4-di(γ-hydroxypropoxy)chlorobenzene or their addition salts with an inorganic acid.

**4.** The dyeing composition according to Claim 2 or 3, characterised in that it contains 0.05 to 3.5% by weight of compound (I') or of one of its addition salts with an acid, based on the total weight of the composition.

**5.** The dyeing composition according to any one of Claims 2 to 4, characterised in that the para-type oxidation dye precursor is chosen from para-phenylenediamines, para-aminophenols, para-heterocyclic compounds or their mixtures.

**6.** The dyeing composition according to Claim 5, characterised in that the para-phenylenediamines correspond to the formula:

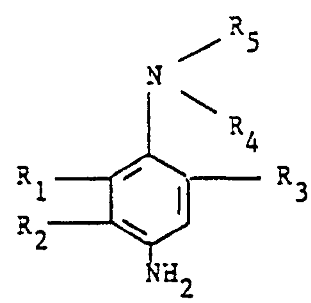

(VIII)

in which
$R_1$, $R_2$ and $R_3$ are identical or different and represent a hydrogen or halogen atom, an alkyl radical having 1 to 4 carbon atoms or an alkoxy radical having 1 to 4 carbon atoms, and
$R_4$ and $R_5$ are identical or different and represent a hydrogen atom, an alkyl, hydroxyalkyl, alkoxyalkyl, carbamylalkyl, mesylaminoalkyl, acetylaminoalkyl, ureidoalkyl, carbethoxyaminoalkyl, piperidinoalkyl or morpholinoalkyl radical, the alkyl or alkoxy groups representd by $R_4$ and $R_5$ having from 1 to 4 carbon atoms, or
$R_4$ and $R_5$, together with the nitrogen atom to which they are bonded, form a piperidino or morpholino heterocycle with the proviso that $R_1$ or $R_3$ represents a hydrogen atom when $R_4$ and $R_5$ do not

represent a hydrogen atom, or are the salts of compounds of formula (VIII).

7. The dyeing composition according to Claim 6, characterised in that it contains at least one para-phenylenediamine chosen from p-phenylenediamine, isopropyl-p-phenylenediamine, p-toluylenediamine, methoxy-para-phenylenediamine, chloro-para-phenylenediamine, 2,6-dimethyl-para-phenylenediamine, 2,3-dimethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di-(β-hydroxyethyl)aniline, 4-amino-N-ethyl-N-(carbamylmethyl)aniline, 3-methyl-4-amino-N-ethyl-N-(carbamylmethyl)aniline, 4-amino-N-ethyl-N-(β-piperidinoethyl)aniline, 3-methyl-4-amino-N-ethyl-N-(β-piperidinoethyl)aniline, 4-amino-N-ethyl-N-(β-morpholinoethyl)aniline, 3-methyl-4-amino-N-ethyl-N-(β-morpholinoethyl)aniline, 4-amino-N-ethyl-N-(β-acetylaminoethyl)aniline, 4-amino-N-(β-methoxyethyl)-aniline, 3-methyl-4-amino-N-ethyl-N-(β-acetylaminoethyl)aniline,4-amino-N-ethyl-N-(β-mesylaminoethyl)-aniline, 3-methyl-4-amino-N-ethyl-N-(β-mesylaminoethyl)aniline, 4-amino-N-ethyl-N-(β-sulphoethyl)-aniline, 3-methyl-4-amino-N-ethyl-N-(β-sulphoethyl)aniline, N-[(4'-amino)phenyl]morpholine and N-[(4'-amino)phenyl]piperidinein the form of a free base or in the form of a cosmetically acceptable salt.

8. The dyeing composition according to Claim 2, characterised in that it contains at least one compound of formula (I') in which $R_2$ is other than a hydrogen atom, or one of its salts with an acid, as coupler, in combination with at least one para-phenylenediamine as defined in Claim 7.

9. The dyeing composition according to Claim 2, characterised in that it contains at least one compound of formula (I') in which $R_2$ is a hydrogen atom, or one of its salts with an acid, as coupler, in combination with at least one para-phenylenediamine as defined in Claim 7.

10. The dyeing composition according to Claim 5, characterised in that it contains at least one para-aminophenol chosen from p-aminophenol, 2-methyl-4-amino-phenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

11. The dyeing composition according to Claim 5, characterised in that the para-type oxidation dye precursor is a para heterocyclic compound chosen from 2,5-diaminopyridine, 2-hydroxy-5-aminopyridine and tetraaminopyrimidine.

12. The dyeing composition according to any one of Claims 2 to 11, characterised in that it contains other couplers chosen from meta-diphenols, meta-aminophenols, meta-phenylenediamines, meta-acylaminophenols, meta-ureidophenols, meta-carbalkoxyaminophenols, α-naphthol, β-ketone compounds and pyrazolones.

13. The dyeing composition according to any one of Claims 2 to 12, characterised in that its total concentration of para-type oxidation dye precursors and couplers is between 0.1 and 7% by weight.

14. The dyeing composition according to any one of Claims 2 to 13, characterised in that it also contains ortho-type dye precursors chosen from ortho-aminophenols, ortho-phenylenediamines and ortho-diphenols.

15. The dyeing composition according to any one of Claims 2 to 14, characterised in that it also contains direct dyes chosen from azo and anthraquinone dyes and the nitro derivatives of the benzene series.

16. The dyeing composition according to any one of Claims 2 to 15, characterised in that it has a pH of between 8 and 11 and preferably of between 9 and 11.

17. The dyeing composition according to any one of Claims 2 to 16, characterised in that it contains 1 to 40% by weight of an organic solvent chosen from lower alkanols, glycerol, glycols or glycol ethers, and their mixtures.

18. The dyeing composition according to any one of Claims 2 to 17, characterised in that it also contains 0.5 to 40% by weight of at least one anionic, cationic, nonionic or amphoteric surfactant or their mixtures.

19. The dyeing composition according to any one of Claims 2 to 18, characterised in that it also contains cosmetic adjuvants chosen from thickeners, antioxidants, penetration agents, sequestering agents, buffers, perfumes, alkalinising agents and propellants.

20. A method for dyeing hair in accordance with a method using development by an oxidant, characterised in that it consists in mixing the tinctorial composition according to any one of Claims 2 to 19, at the time of use, with an oxidising solution in a sufficient amount, then applying the mixture obtained to the hair, leaving said mixture on the hair for 10 to 40 minutes, preferably 15 to 30 minutes, then rinsing the hair, washing it with shampoo, rinsing it again and drying it.

21. The method for dyeing hair in accordance with a method using development by an oxidant, characterised in that it consists in applying to the hair, in a first step, a dyeing composition containing at least one para-type oxidation dye precursor as defined in one of Claims 5 to 11, then, in a second step, applying a dyeing composition containing a compound of formula (I') or one of its salts, the oxidising agent being present in the composition applied in the second step or being applied to the hair itself in a third step, leaving the composition on the hair for 10 to 40 minutes, preferably 15 to 30 minutes, then rinsing the hair, washing it with shampoo, rinsing it again and drying it.

22. Process for the preparation of a composition for dyeing hair, characterised in that it consists in dissolving, in an aqueous, cosmetically acceptable carrier, as a coupler, 0.05 to 3.5% by weight of at least one compound of formula (I')

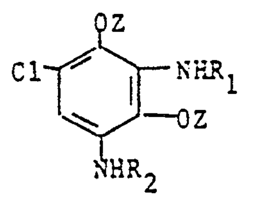

(I')

in which
Z and Z' independently of one another represent an alkyl radical having from 1 to 4 carbon atoms or a hydroxyalkyl radical having from 2 to 4 carbon atoms, and
$R_1$ and $R_2$ independently of one another represent an alkyl radical having from 1 to 4 carbon atoms or a mono-or polyhydroxyalkyl radical having 2 or 3 carbon atoms, or an addition salt of this compound with an acid, this amount being calculated on the basis of the total weight of the composition, and at least one para-type oxidation dye precursor, the total concentration of couplers and para-type oxidation dye precursors being between 0.1 and 7% by weight based on the total weight of the composition, then adding at least one cosmetic adjuvant chosen from anionic, cationic, nonionic or amphoteric surfactants or their mixtures, organic solvents, thickeners, antioxidants, penetration agents, sequestering agents, buffers and perfumes, and finally adjusting the pH of the dyeing composition to a value of between 8 and 11, and preferably between 9 and 11, with the aid of an alkalinising agent.

23. Process according to Claim 22, characterised in that the coupler used is at least one compound chosen from 5-(β-hydroxyethyl)amino-3-amino-2,4-dimethoxychlorobenzene, 5-amino-3-(β-hydroxyethyl)amino-2,4-dimethoxychlorobenzene,3,5-di(β-hydroxyethyl)amino-2,4-dimethoxychlorobenzene, 5-methylamino-3-amino-2,4-dimethoxychlorobenzene, 3,5-diamino-2,4-dimethoxychlorobenzene, 3,5-diamino-2,4-diethoxychlorobenzene, 3,5-diamino-2,4-di(γ-hydroxypropoxy)chlorobenzene or their addition salts with an inorganic acid.

24. Process according to Claim 22 or 23, characterised in that a para-type oxidation dye precursor chosen from para-phenylenediamines, para-aminophenols, para-heterocyclic compounds or their mixtures is used.

**25.** Process according to Claim 24, characterised in that a para-phenylenediamine is used which corresponds to the formula:

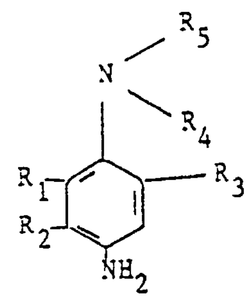

(VIII)

in which
$R_1$, $R_2$ and $R_3$ are identical or different and represent a hydrogen or halogen atom, an alkyl radical having 1 to 4 carbon atoms or an alkoxy radical having 1 to 4 carbon atoms, and
$R_4$ and $R_5$ are identical or different and represent a hydrogen atom, an alkyl, hydroxyalkyl, alkoxyalkyl, carbamylalkyl, mesylaminoalkyl, acetylaminoalkyl, ureidoalkyl, carbethoxyaminoalkyl, piperidinoalkyl or morpholinoalkyl radical, the alkyl or alkoxy groups representd by $R_4$ and $R_5$ having from 1 to 4 carbon atoms, or
$R_4$ and $R_5$, together with the nitrogen atom to which they are bonded, form a piperidino or morpholino heterocycle with the proviso that $R_1$ or $R_3$ represents a hydrogen atom when $R_4$ and $R_5$ do not represent a hydrogen atom, or a salt of a compound of formula (VIII).

**26.** Process according to Claim 25, characterised in that a para-phenylenediamine is used which is chosen from p-phenylenediamine, isopropyl-p-phenylenediamine, p-toluylenediamine, methoxy-para-phenylenediamine, chloro-para-phenylenediamine, 2, 6-dimethyl-para-phenylenediamine, 2,3-dimethyl-para-phenylenediamine, 2,5-dimethyl-para-phenylenediamine, 2-methyl-5-methoxy-para-phenylenediamine, 2, 6-dimethyl-5-methoxy-para-phenylenediamine, N,N-dimethylpara-phenylenediamine, 3-methyl-4-amino-N,N-diethylaniline,N,N-di(β-hydroxyethyl)-para-phenylenediamine, 3-methyl-4-amino-N,N-di(β-hydroxyethyl)aniline, 3-chloro-4-amino-N,N-di-(β-hydroxyethyl)aniline, 4-amino-N-ethyl-N-(carbamylmethyl)aniline, 3-methyl-4-amino-N-ethyl-N-(carbamylmethyl)aniline, 4-amino-N-ethyl-N-(β-piperidinoethyl)aniline, 3-methyl-4-amino-N-ethyl-N-(β-piperidinoethyl)aniline, 4-amino-N-ethyl-N-(β-morpholinoethyl)aniline, 3-methyl-4-amino-N-ethyl-N-(β-morpholinoethyl)aniline, 4-amino-N-ethyl-N-(β-acetylaminoethyl)aniline, 4-amino-N-β-methoxyethylaniline, 3-methyl-4-amino-N-ethyl-N-(β-acetylaminoethyl)aniline, 4-amino-N-ethyl-N-(β-mesylaminoethyl)aniline, 3-methyl-4-amino-N-ethyl-N-(β-mesylaminoethyl)aniline, 4-amino-N-ethyl-N-(β-sulphoethyl)aniline, 3-methyl-4-amino-N-ethyl-N-(β-sulphoethyl)aniline, N-[(4'-amino)phenyl]morpholine and N-[(4'-amino)phenyl]piperidine in the form of a free base or in the form of a cosmetically acceptable salt.

**27.** Process according to Claim 22, characterised in that a compound of formula (I') in which $R_2$ is other than a hydrogen atom, or one of its salts with an acid, is used as coupler, in combination with at least one para-phenylenediamine as defined in Claim 26.

**28.** Process according to Claim 22, characterised in that a compound of formula (I') in which $R_2$ is a hydrogen atom, or one of its salts with an acid, is used as coupler, in combination with at least one para-phenylenediamine as defined in Claim 26.

**29.** Process according to Claim 24, characterised in that a para-aminophenol is used which is chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

**30.** Process according to Claim 24, characterised in that a para heterocyclic compound chosen from 2,5-diaminopyridine, 2-hydroxy-5-aminopyridine and tetraaminopyrimidine is used as para-type oxidation dye precursor.

**31.** Process according to any one of Claims 22 to 30, characterised in that other couplers chosen from meta-diphenols, meta-aminophenols, meta-phenylenediamines, meta-acylaminophenols, meta-ureidophenols, meta-carbalkoxyaminophenols, α-naphthol, β-ketone compounds and pyrazolones are added.

**32.** Process according to any one of Claims 22 to 31, characterised in that ortho-type dye precursors chosen from ortho-aminophenols, ortho-phenylenediamines and ortho-diphenols are also added.

**33.** Process according to any one of Claims 22 to 32, characterised in that 1 to 40% by weight of an organic solvent chosen from lower alkanols, glycerol, glycols or glycol ethers, and their mixtures, are added.

**34.** Process according to any one of Claims 22 to 33, characterised in that 0.5 to 40% by weight of at least one anionic, cationic, nonionic or amphoteric surfactant or their mixtures, are added.

**Patentansprüche**
**Patentansprüche für folgende Vertragsstaaten : DE, GB, FR, AT, SE, IT, NL, CH, BE, GR, LI**

**1.** Verbindungen gemäß der Formel:

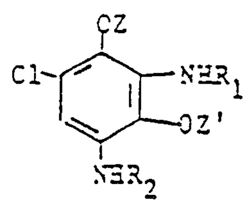

(I)

worin Z und Z' unabhängig voneinander einen Alkylrest mit 1 bis 4 Kohlenstoffatomen oder einen Hydroxyalkylrest mit 2 bis 4 Kohlenstoffatomen und $R_1$ und $R_2$ unabhängig voneinander ein Wasserstoffatom, einen Alkylrest mit 1 bis 4 Kohlenstoffatomen oder einen Mono- oder Polyhydroxyalkylrest mit 2 bis 3 Kohlenstoffatomen darstellen, unter der Bedingung, daß, wenn $R_1$ und $R_2$ gleichzeitig ein Wasserstoffatom bezeichnen, Z und Z' nicht gleichzeitig den Methylrest bezeichnen, sowie deren Additionssalze mit einer Säure.

**2.** Verbindungen gemäß Anspruch 1,
dadurch **gekennzeichnet**, daß
sie aus
3-(beta-Hydroxyethyl)amino-5-amino-2,4-dimethoxychlorbenzol, 3-Amino-5(beta-hydroxyethyl)amino-2,4-dimethoxychlorbenzol, 3,5-Di(beta-hydroxyethyl)amino-2,4-dimethoxychlorbenzol, 3-Methylamino-5-amino-2,4-dimethoxychlorbenzol, 3,5-Diamino-2,4-diethoxychlorbenzol, 3,5-Diamino-2,4-di(gamma-hydroxypropoxy)chlorbenzol sowie aus deren Mineralsäureadditionssalzen ausgewählt sind.

**3.** Verwendung einer Verbindung der Formel (I'):

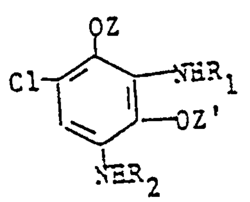

(I')

worin Z und Z' unabhängig voneinander einen Alkylrest mit 1 bis 4 Kohlenstoffatomen oder Hydroxyalkylrest mit 2 bis 4 Kohlenstoffatomen und $R_1$ und $R_2$ unabhängig voneinander ein Wasserstoffatom, einen Alkylrest mit 1 bis 4 Kohlenstoffatomen oder Mono- oder Polyhydroxyalklrest mit 2 bis 3 Kohlenstoffatomen darstellen, oder eines Säureadditionssalzes dieser Verbindung als Kuppler für die Oxidationsfärbung von Keratinfasern und insbesondere menschlicher Haare.

4. Kapillarfärbezusammensetzung,
dadurch **gekennzeichnet,** daß
sie in einem wässrigen, kosmetisch verträglichen Träger als Kuppler mindestens eine Verbindung der Formel (I') gemäß Anspruch 3 oder eines ihrer Säureadditionssalze zusammen mit mindestens einer Oxidationsfarbstoffvorstufe vom para-Typ enthält.

5. Färbezusammensetzung gemäß Anspruch 4,
dadurch **gekennzeichnet,** daß
sie als Kuppler eine Verbindung enthält, die aus 5-(beta-Hydroxyethyl)amino-3-amino-2,4-dimethoxychlorbenzol, 5-Amino-3(beta-hyroxyethyl)amino-2,4-dimethoxychlorbenzol, 3,5-Di(beta-hydroxyethyl)-amino -2, 4-dimethoxychlorbenzol, 5-Methylamino-3-amino-2,4-dimethoxychlorbenzol, 3,5-Diamino-2,4-dimethoxychlorbenzol, 3,5-Diamino-2,4-diethoxychlorbenzol, 3,5-Diamino-2,4-di(gamma-hydroxypropoxy)chlorbenzol oder aus deren Mineralsäureadditionssalzen ausgewählt ist.

6. Färbezusammensetzung gemäß Anspruch 4 oder 5,
dadurch **gekennzeichnet,** daß
sie 0,05 bis 3,5 Gewichtsprozent der Verbindung (I') oder eines ihrer Säureadditionssalze, bezogen auf das Gesamtgewicht der Zusammensetzung, enthält.

7. Färbezusammensetzung gemäß jedem der Ansprüche 4 bis 6,
dadurch **gekennzeichnet,** daß
die Oxidationsfarbstoffvorstufe vom para-Typ aus p-Phenylendiaminen, p-Aminophenolen, p-substituierten Heterozyklen oder deren Mischungen ausgewählt ist.

8. Färbezusammensetzung gemäß Anspruch 7, dadurch gekennzeichnet, daß die p-Phenylendiamine der Formel entsprechen:

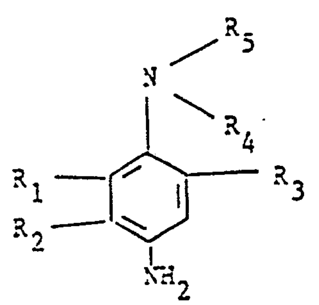

(VIII)

worin $R_1$, $R_2$ und $R_3$ gleich oder verschieden sind und ein Wasserstoff- oder Halogenatom, einen Alkylrest mit 1 bis 4 Kohlenstoffatomen, einen Alkoxyrest mit 1 bis 4 Kohlenstoffatomen dartellen und $R_4$ und $R_5$ gleich oder verschieden sind und ein Wasserstoffatom, einen Alkyl-, Hydroxyalkyl, Alkoxyalkyl-, Carbamylalkyl-, Mesylaminoalkyl-, Acetylaminoalkyl-, Ureidoalkyl-, Carbethoxyaminoalkyl-, Piperidinoalkyl-, Morpholinoalkylrest darstellen, wobei die durch $R_4$ und $R_5$ dargestellten Alkyl- oder Alkoxygruppen 1 bis 4 Kohlenstoffatome aufweisen, oder $R_4$ und $R_5$ zusammen mit dem Stickstoffatom, an das sie gebunden sind, einen Piperidin- oder Morpholinheterozyklus bilden, unter der Maßgabe, daß $R_1$ oder $R_3$ ein Wasserstoffatom darstellen , wenn $R_4$ und $R_5$ kein Wasserstoffatom darstellen , oder daß die p-Phenylendiamine Salze der Verbindungen der Formel (VIII) sind.

9. Färbezusammensetzung gemäß Anspruch 8,
dadurch **gekennzeichnet,** daß
sie mindestens ein p-Phenylendiamin enthält, ausgewählt aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, Isopropyl-p-phenylendiamin, 2,3-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(beta-hydroxyethyl)-p-phenylendiamin, 3-Methyl-4-amino-N,-di-(beta-hydroxyethyl)-anilin, 3-Chlor-4-amino-N,N-di-(beta-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl, beta-piperidinoethyl)anilin, 3-Methyl-4-amino-N,N-(ethyl, beta-piperidinoethyl)anilin, 4-Amino-N,N-(ethyl,

beta-morpholinoethyl)anilin, 3-Methyl-4-amino-N,N-(ethyl, beta-morpholinoethyl)anilin, 4-Amino-N,N-(ethyl, beta-acetylaminoethyl)anilin, 4-Amino-N-beta-methoxyethylanilin, 3-Methyl-4-amino-N,N-(ethyl,beta-acetylaminoethyl)anilin, 4-Amino-N,N-(ethyl, beta-mesylaminoethyl)anilin, 3-Methyl-4-amino-N,N-(ethyl, beta-mesylaminoethyl)anilin, 4-Amino-N,N-(ethyl, beta-sulfoethyl)anilin, 3-Methyl-4-amino-N,N-(ethyl, beta-sulfoethyl)anilin, N((4'-Amino)phenyl)morpholin, N-((4'Amino)phenyl)piperidin, in Form der freien Base oder eines kosmetisch verträglichen Salzes.

**10.** Färbezusammensetzung gemäß Anspruch 9,
dadurch **gekennzeichnet,** daß
sie als Kuppler mindestens eine Verbindung der Formel (I'), worin $R_2$ von einem Wasserstoffatom verschieden ist, oder eines ihrer Salze mit einer Säure zusammen mit mindestens einem wie in Anspruch 9 definierten p-Phenylendiamin enthält.

**11.** Färbezusammensetzung gemäß Anspruch 9,
dadurch **gekennzeichnet,** daß
sie als Kuppler mindestens eine Verbindung der Formel (I'), worin $R_2$ ein Wassertoffatom ist, oder eines ihrer Salze mit einer Säure zusammen mit mindestens einem wie in Anspruch 9 definierten p-Phenylendiamin enthält.

**12.** Färbezusammensetzung gemäß Anspruch 7,
dadurch **gekennzeichnet,** daß
sie mindestens ein p-Aminophenol enthält, ausgewählt aus p-Aminophenol, 2-Methyl-4-aminophenol, 3-Methyl-4-aminophenol, 2-Chlor-4-aminophenol, 3-Chlor-4-aminophenol, 2-6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(beta-Hydroxyethyl)-4-aminophenol, 2-Methoxy-4-aminophenol, 3-Methoxy-4-aminophenol.

**13.** Färbezusammensetzung nach Anspruch 7,
dadurch **gekennzeichnet,** daß die Oxidationsfarbstoffvorstufe vom para-Typ eine p-substituierte heterozyklische Verbindung ist, ausgewählt aus 2,5-Diaminopyridin, 2-Hydroxy-5-aminopyridin und Tetraaminopyrimidin.

**14.** Färbezusammensetzung gemäß jedem der Ansprüche 4 bis 13,
dadurch **gekennzeichnet,** daß
sie weitere Kuppler enthält, ausgewählt aus m-Diphenolen, m-Aminophenolen, m-Phenylendiaminen, m-Acylaminophenolen, m-Ureidophenolen, m-Carbalkoxyaminophenolen, alpha-Naphthol, beta-Ketoverbindungen und Pyrazolonen.

**15.** Färbezusammensetzung gemäß jedem der Ansprüche 4 bis 14,
dadurch **gekennzeichnet,** daß
ihre Gesamtkonzentration an Oxidationsfarbstoffvorstufen vom para-Typ und an Kupplern zwischen 0,1 und 7 Gewichtsprozent liegt.

**16.** Färbezusammensetzung gemäß jedem der Ansprüche 4 bis 15,
dadurch **gekennzeichnet,** daß
sie zusätzlich Farbstoffvorstufen vom ortho-Typ enthält, ausgewählt aus o-Aminophenolen, o-Phenylendiaminen und o-Diphenolen.

**17.** Färbezusammensetzung gemäß jedem der Ansprüche 4 bis 16,
dadurch **gekennzeichnet,** daß
sie zusätzlich Direktfarbstoffe enthält, ausgewählt aus Azofarbstoffen, Antrachinonfarbstoffen und nitrierten Derivaten der Benzolreihe.

**18.** Färbezusammensetzung gemäß jedem der Ansprüche 4 bis 17,
dadurch **gekennzeichnet,** daß
sie einen pH zwischen 8 und 11, vorzugsweise zwischen 9 und 11, aufweist.

**19.** Färbezusammensetzung gemäß jedem der Ansprüche 4 bis 18,

dadurch **gekennzeichnet,** daß
sie 1 bis 40 Gewichtsprozent eines organischen Lösungsmittels enthält, ausgewählt aus Niedrigalkanolen, Glyzerin, Glycolen oder Etherglycolen sowie deren Mischungen.

**20.** Färbezusammensetzung gemäß jedem der Ansprüche 4 bis 19,
dadurch **gekennzeichnet,** daß
sie zusätzlich 0,5 bis 40 Gewichtsprozent mindestens eines anionischen, kationischen, nicht-ionischen, amphoteren oberflächenaktiven Mittels oder von deren Mischungen enthält.

**21.** Färbezusammensetzung gemäß jedem der Ansprüche 4 bis 20,
dadurch **gekennzeichnet,** daß
sie zusätzlich kosmetische Hilfstoffe enthält, ausgewählt aus Verdickern, Antioxidantien, Penetrations-, Sequestriermitteln, Puffer , Parfümen, alkalisch machenden Mitteln, Treibmitteln.

**22.** Verfahren zur Haarfärbung , wobei man die Entwicklung durch ein Oxidationsmittel anwendet,
dadurch **gekennzeichnet,** daß
man zum Zeitpunkt der Anwendung die Färbezusammensetzung gemäß jedem der Ansprüche 4 bis 21 mit einer oxidierenden Lösung in hinreichender Menge vermischt, anschließend die Mischung auf die Haare aufbringt, sie während 10 bis 40 Minuten, vorzugsweise 15 bis 30 Minuten, darauf verweilen läßt, danach die Haare spült, sie durch Schamponieren wäscht, erneut spült und trocknet.

**23.** Verfahren zur Haarfärbung , wobei man die Entwicklung durch ein Oxidationsmittel anwendet,
dadurch **gekennzeichnet,** daß
man zuerst auf die Haare eine Färbezusammensetzung aufbringt, enthaltend mindestens eine Oxidationsfarbstoffvorstufe vom para-Typ, wie in einem der Ansprüche 7 bis 13 definiert, anschließend in einer zweiten Stufe eine Färbezusammensetzung, enthaltend eine Verbindung der Formel (I') oder eines ihrer Salze, aufbringt, wobei das Oxidationsmittel in der in der zweiten Stufe aufgebrachten Zusammensetzung vorliegt oder auch auf die Haare selbst in einer dritten Stufe aufgebracht wird, während 10 bis 40 Minuten, vorzugsweise 15 bis 30 Minuten, verweilen läßt, dann die Haare spült, sie durch Schamponieren wäscht, erneut spült und trocknet.

**Patentansprüche für folgenden Vertragsstaat : ES**

**1.** Verwendung einer Verbindung der Formel (I')

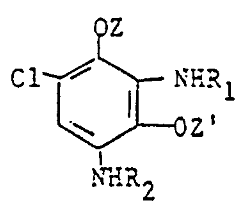

(I')

worin Z und Z' unabhängig voneinander einen Alkylrest mit 1 bis 4 Kohlenstoffatomen oder Hydroxyalkylrest mit 2 bis 4 Kohlenstoffatomen und $R_1$ und $R_2$ unabhängig voneinander ein Wasserstoffatom, einen Alkylrest mit 1 bis 4 Kohlenstoffatomen oder einen Mono- oder Polyhydroxyalkylrest mit 2 oder 3 Kohlenstoffatomen darstellen, oder eines Säureadditionssalzes dieser Verbindung als Kuppler für die Oxidationsfärbung von Keratinfasern und insbesondere menschlicher Haare.

**2.** Kapillarfärbezusammensetzung, dadurch gekennzeichnet, daß sie in einem wässrigen, kosmetisch verträglichen Träger mindestens eine Verbindung der Formel (I') gemäß Anspruch 1 oder eines ihrer Säureadditionssalze als Kuppler zusammen mit mindestens einer Oxidationsfarbstoffvorstufe vom para-Typ enthält.

**3.** Färbezusammensetzung gemäß Anspruch 2, dadurch gekennzeichnet, daß die als Kuppler eine Verbindung enthält, ausgewählt aus 5-(beta-Hydroxyethyl) amino-3-amino-2 4-dimethoxychlorbenzol, 5-Amino-3-(beta-hyroxyethyl)amino-2,4-dimethoxychlorbenzol, 3,5-Di(beta-hydroxyethyl)amino-2,4-dimethoxychlorbenzol, 5-Methylamino-3-amino-2,4-dimethoxychlorbenzol, 3,5-Diamino-2,4-dimethoxychlorbenzol, 3,5-Diamino-2,4 diethoxychlorbenzol, 3,5-Diamino-2,4-di(gamma-hydroxypropoxy)chlorbenzol oder aus deren Mineralsäureadditionssalzen.

4. Färbezusammensetzung gemäß Anspruch 2 oder 3, dadurch gekennzeichnet, daß sie 0,05 bis 3,5 Gewichtsprozent der Verbinduang (I') oder eines ihrer Säureadditionssalze, bezogen auf das Gesamtgewicht der Zusammensetzung, enthält.

5. Färbezusammensetzung gemäß jedem der Ansprüche 2 bis 4, dadurch gekennzeichnet, daß die Oxidationsfarbstoffvorstufe vom para-Typ aus p-Phenylendiaminen, p-Aminophenolen, p-substituierten Heterozyklen oder deren Mischungen ausgewählt ist.

6. Färbezusammensetzung gemäß Anspruch 5, dadurch gekennzeichnet, daß die p-Phenylendiamine der Formel entsprechen:

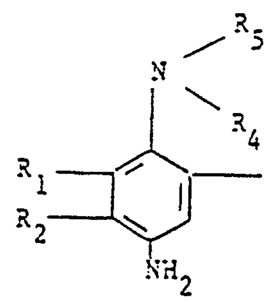

(VIII)

worin $R_1$, $R_2$ und $R_3$ gleich oder verschieden sind und ein Wasserstoff- oder Halogenatom, einen Alkylrest mit 1 bis 4 Kohlenstoffatomen, einen Alkoxyrest mit 1 bis 4 Kohlenstoffatomen dartellen und $R_4$ und $R_5$ gleich oder verschieden sind und ein Wasserstoffatom, einen Alkyl-, Hydroxyalkyl, Alkoxyalkyl-, Carbamylalkyl-, Mesylaminoalkyl-, Acetylaminoalkyl-, Ureidoalkyl-, Carbethoxyaminoalkyl-, Piperidinoalkyl-, Morpholinoalkylrest darstellen, wobei die durch $R_4$ und $R_5$ dargestellten Alkyl- oder Alkoxygruppen 1 bis 4 Kohlenstoffatome aufweisen, oder $R_4$ und $R_5$ zusammen mit dem Stickstoffatom, an das sie gebunden sind, einen Piperidin- oder Morpholinheterozyklus bilden, unter der Maßgabe, daß $R_1$ oder $R_3$ ein Wasserstoffatom dartellen, wenn $R_4$ und $R_5$ kein Wasserstoffatom dartellen, oder daß die p-Phenylendiamine Salze der Verbindungen der Formel (VIII) sind.

7. Färbezusammensetzung gemäß Anspruch 6, dadurch gekennzeichnet, daß sie mindestens ein p-Phenylendiamin enthält, ausgewählt aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, Isopropyl-p-phenylendiamin, 2,3-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(beta-hydroxyethyl)p-phenylendiamin, 3-Methyl-4-amino-N,N-di-(beta-hydroxyethyl)-anilin, 3-Chlor-4-amino-N,N-di-(beta-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl, beta-piperidinoethyl)anilin, 3-Methyl-4-amino-N,N-(ethyl, beta-piperidinoethyl)anilin, 4-Amino-N,N-(ethyl, beta-morpholinoethyl)anilin, 3-Methyl-4-amino-N,N-(ethyl, beta-morpholinoethyl)anilin, 4-Amino-N,N-(ethyl, beta-acetylaminoethyl)-anilin, 4-Amino-N-beta-methoxyethylanilin, 3-Methyl-4-amino-N,N-(ethyl,beta-acetylaminoethyl)anilin , 4-Amino-N,N-(ethyl, beta-mesylaminoethyl)anilin, 3-Methyl-4-amino-N,N-(ethyl, beta-mesylaminoethyl)-anilin, 4-Amino-N,N-(ethyl, beta-sulfoethyl)anilin, 3-Methyl-4-amino-N,N-(ethyl, beta-sulfoethyl)anilin, N-(-(4'-Amino)phenyl)morpholin, N-((4'-Amino)phenyl)piperidin, in Form der freien Base oder eines kosmetisch verträglichen Salzes.

8. Färbezusammensetzung gemäß Anspruch 2, dadurch gekennzeichnet, daß sie als Kuppler mindestens eine Verbindung der Formel (I'), worin $R_2$ von einem Wasserstoffatom verschieden ist, oder eines ihrer Salze mit einer Säure zusammen mit mindestens einem wie in Anspruch 7 definierten p-Phenylendiamin enthält.

9. Färbezusammensetzung gemäß Anspruch 2, dadurch gekennzeichnet, daß sie als Kuppler mindestens eine Verbindung der Formel (I'), worin $R_2$ ein Wassertoffatom ist, oder eines ihrer Salze mit einer Säure

zusammen mit mindestens einem wie in Anspruch 7 definierten p-Phenylendiamin enthält.

10. Färbezusammensetzung gemäß Anspruch 5, dadurch gekennzeichnet, daß sie mindestens ein p-Aminophenol enthält, ausgewählt aus p-Aminophenol, 2-Methyl-4-aminophenol, 3-Methyl-4-aminophenol, 2-Chlor-4-aminophenol, 3-Chlor-4-aminophenol, 2-6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(beta-Hydroxyethyl)-4-aminophenol, 2-Methoxy-4-aminophenol, 3-Methoxy-4-aminophenol.

11. Färbezusammensetzung nach Anspruch 5, dadurch gekennzeichnet, daß die Oxidationsfarbstoffvorstufe vom para-Typ eine p-substituierte heterozyklische Verbindung ist, ausgewählt aus 2,5-Diaminopyridin, 2-Hydroxy-5-aminopyridin und Tetraaminopyrimidin.

12. Färbezusammensetzung gemäß jedem der Ansprüche 2 bis 11, dadurch gekennzeichnet, daß sie weitere Kuppler enthält, ausgewählt aus m-Diphenolen, m-Aminophenolen, m-Phenylendiaminen, m-Acylaminophenolen, m-Ureidophenolen, m-Carbalkoxyaminophenolen, alpha-Naphthol, beta-Ketoverbindungen und Pyrazolonen.

13. Färbezusammensetzung gemäß jedem der Ansprüche 2 bis 12, dadurch gekennzeichnet, daß ihre Gesamtkonzentration an oxidationsfarbstoffvorstufen vom para-Typ und an Kupplern zwischen 0,1 und 7 Gewichtsprozent liegt.

14. Färbezusammensetzung gemäß jedem der Ansprüche 2 bis 13, dadurch gekennzeichnet, daß sie zusätzlich Farbstoffvorstufen vom ortho-Typ enthält, ausgewählt aus o-Aminophenolen, o-Phenylendiaminen und o-Diphenolen.

15. Färbezusammensetzung gemäß jedem der Ansprüche 2 bis 14, dadurch gekennzeichnet, daß sie zusätzlich Direktfarbstoffe enthält, ausgewählt aus Azofarbstoffen, Antrachinonfarbstoffen und nitrierten Derivaten der Benzolreihe.

16. Färbezusammensetzung gemäß jedem der Ansprüche 2 bis 15, dadurch gekennzeichnet, daß sie einen pH zwischen 8 und 11, vorzugsweise zwischen 9 und 11, aufweist.

17. Färbezusammensetzung gemäß jedem der Ansprüche 2 bis 16, dadurch gekennzeichnet, daß sie 1 bis 40 Gewichtsprozent eines organischen Lösungsmittels enthält, ausgewählt aus Niedrigalkanolen, Glyzerin, Glycolen oder Etherglycolen sowie deren Mischungen.

18. Färbezusammensetzung gemäß jedem der Ansprüche 2 bis 17, dadurch gekennzeichnet, daß sie zusätzlich 0,5 bis 40 Gewichtsprozent mindestens eines anionischen, kationischen, nicht-ionischen, amphoteren oberflächenaktiven Mittels oder von deren Mischungen enthält.

19. Färbezusammensetzung gemäß jedem der Ansprüche 2 bis 18, dadurch gekennzeichnet, daß sie zusätzlich kosmetische Hilfsstoffe enthält, ausgewählt aus Verdickern, Antioxidantien, Penetrations-, Sequestriermitteln, Tampons, Parfumen, alkalisch machenden Mitteln, Treibmitteln.

20. Verfahren zur Haarfärbung , wobei man die Entwicklung durch ein Oxidationsmittel anwendet, dadurch gekennzeichnet, daß man zum Zeitpunkt der Anwendung die Färbezusammensetzung gemäß jedem der Ansprüche 2 bis 19 mit einer oxidierenden Lösung in hinreichender Menge vermischt, anschließend die Mischung auf die Haare aufbringt, sie während 10 bis 40 Minuten, vorzugsweise 15 bis 30 Minuten, darauf verweilen läßt, danach die Haare spült, sie durch Schamponieren wäscht, erneut spült und trocknet.

21. Verfahren zur Haarfärbung , wobei man die Entwicklung durch ein Oxidationsmittel anwendet, dadurch gekennzeichnet, daß man zuerst auf die Haare eine Färbezusammensetzung aufbringt, enthaltend mindestens eine Oxidationsfarbstoffvorstufe vom para-Typ, wie in einem der Ansprüche 5 bis 11 definiert, anschließend in einer zweiten Stufe eine Färbezusammensetzung, enthaltend eine Verbindung der Formel (I') oder eines ihrer Salze, aufbringt, wobei das Oxidationsmittel in der in der zweiten Stufe aufgebrachten Zusammensetzung vorliegt oder auch auf die Haare selbst in einer dritten Stufe aufgebracht wird, während 10 bis 40 Minuten, vorzugsweise 15 bis 30 Minuten, verweilen läßt, dann die

Haare spült, sie durch Schamponieren wäscht, erneut spült und trocknet.

**22.** Verfahren zur Herstellung einer Haarfärbezusammensetzung , dadurch gekennzeichnet, daß man in einem wässrigen, kosmetisch verträglichen Träger als Kuppler 0,05 bis 3,5 Gewichtsprozent mindestens einer Verbindung der Formel (I')

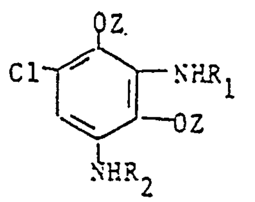

(I')

worin Z und Z' unabhängig voneinander einen Alkylrest mit 1 bis 4 Kohlenstoffatomen oder Hydroxyalkylrest mit 2 bis 4 Kohlenstoffatomen und $R_1$ und $R_2$ unabhängig voneinander einen Alkylrest mit 1 bis 4 Kohlenstoffatomen oder einen Mono- oder Polyalkylrest mit 2 oder 3 Kohlenstoffatomen darstellen, oder eines Säureadditionssalzes dieser Verbindung, wobei diese Menge auf Basis des Gesamtgewichts der Zusamensetzung berechnet ist, sowie mindestens eine Oxidationsfarbstoffvorstufe von para-Typ auflöst, wobei die Gesamtkonzentration an Kupplern und Oxidationsfarbstoffvorstufen vom para-Typ zwischen 0,1 und 7 Gewichtsprozent auf Basis des Gesamtgewichts der Zusammensetzung liegt, anschließend mindestens einen kosmetischen Hilfsstoff zufügt, ausgewählt aus anionischen, kationischen, nicht-ionischen, amphoteren oberflächenaktiven Mitteln oder deren Mischungen, organischen Lösungsmitteln, Verdickern, Antioxidantien, Penetrations-, Sequestriermitteln, Puffer und Parfümen, und schließlich den pH der Färbezusammensetzung mittels eines alkalisch machenden Mittels auf einen Wert zwishen 8 und 11, vorzugsweise zwischen 9 und 11, einstellt.

**23.** Verfahren gemäß Anspruch 22, dadurch gekennzeichnet, daß man als Kuppler mindestens eine Verbindung verwendet, ausgewählt aus 5-(beta-Hydroxyethyl) amino-3-amino-2, 4-dimethoxychlorbenzol, 5-Amino-3(beta-hyroxyethyl)amino-2,4-dimethoxychlorbenzol, 3,5-Di(beta-hydroxyethyl)amino-2,4-dimethoxychlorbenzol, 5-Methylamino-3-amino-2,4-dimethoxychlorbenzol, 3,5-Diamino-2,4-dimethoxychlorbenzol, 3,5-Diamino-2,4-diethoxychlorbenzol, 3,5-Diamino-2,4-di(gamma-hydroxypropoxy)-chlorbenzol oder deren Mineralsäureadditionssalzen.

**24.** Verfahren gemäß Anspruch 22 oder 23, dadurch gekennzeichnet, daß man eine Oxidationsfarbstoffvorstufe vom para-Typ verwendet, die aus p-Phenylendiaminen, p-Aminophenolen, p-substituierten Heterozyklen oder deren Mischungen ausgewählt ist.

**25.** Verfahren gemäß Anspruch 24, dadurch gekennzeichnet, daß man ein p-Phenylendiamin der Formel verwendet:

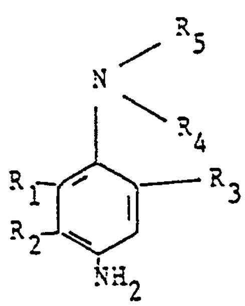

(VIII)

worin $R_1$, $R_2$, und $R_3$ gleich oder verschieden sind und ein Wasserstoff- oder Halogenatom, einen Alkylrest mit 1 bis 4 Kohlenstoffatomen, einen Alkoxyrest mit 1 bis 4 Kohlenstoffatomen dartellen und $R_4$ und $R_5$ gleich oder verschieden sind und ein Wasserstoffatom, einen Alkyl-, Hydroxyalkyl, Alkoxyalkyl-, Carbamylalkyl-, Mesylaminoalkyl-, Acetylaminoalkyl-, Ureidoalkyl-, Carbethoxyaminoalkyl-, Piperidinoalkyl-, Morpholinoalkylrest darstellen, wobei die durch $R_4$ und $R_5$ dargestellten Alkyl- oder Alkoxygruppen 1 bis 4 Kohlenstoffatome aufweisen, oder R4 und $R_5$ zusammen mit dem Stickstoff-

atom, an das sie gebunden sind, einen Piperidin- oder Morpholinheterozyklus bilden, unter der Maßgabe, daß $R_1$ oder $R_3$ ein Wasserstoffatom darstellen, wenn $R_4$ und $R_5$ kein Wasserstoffatom dartellen, oder daß man ein Salz einer Verbindung der Formel (VIII) verwendet.

**26.** Verfahren gemäß Anspruch 25, dadurch gekennzeichnet, daß man ein p-Phenylendiamin verwendet, ausgewählt aus p-Phenylendiamin, p-Toluylendiamin, Methoxy-p-phenylendiamin, Chlor-p-phenylendiamin, 2, 6-Dimethyl-p-phenylendiamin, 2, 5-Dimethyl-p-phenylendiamin, Isopropyl-p-phenylendiamin, 2,3-Dimethyl-p-phenylendiamin, 2-Methyl-5-methoxy-p-phenylendiamin, 2, 6-Dimethyl-5-methoxy-p-phenylendiamin, N,N-Dimethyl-p-phenylendiamin, 3-Methyl-4-amino-N,N-diethylanilin, N,N-Di-(beta-hydroxyethyl)-p-phenylendiamin, 3-Methyl-4-amino-N,N-di-(beta-hydroxyethyl)-anilin, 3-Chlor-4-amino-N,N-di-(beta-hydroxyethyl)anilin, 4-Amino-N,N-(ethyl,carbamylmethyl) anilin, 3-Methyl-4-amino-N,N-(ethyl,carbamylmethyl) anilin, 4-Amino-N,N-(ethyl, beta-piperidinoethyl)anilin, 3-Methyl-4-amino-N,N-(ethyl, beta-piperidinoethyl)anilin, 4-Amino-N,N-(ethyl, beta-morpholinoethyl)anilin, 3-Methyl-4-amino-N,N-(ethyl, beta-morpholinoethyl)anilin, 4-Amino-N,N-(ethyl, beta-acetylaminoethyl) anilin, 4-Amino-N-beta-methoxyethylanilin, 3-Methyl-4-amino-N,N-(Ethyl,beta-acetylaminoethyl) anilin 4-Amino-N,N-(ethyl, beta-mesylaminoethyl)anilin, 3-Methyl-4-amino-N,N-(ethyl, beta-mesylaminoethyl)anilin, 4-Amino-N,N-(ethyl, beta-sulfoethyl)anilin, 3-Methyl-4-amino-N,N-(ethyl, beta-sulfoethyl)anilin, N-((4'-Amino)phenyl)-morpholin, N-((4'-Amino)phenyl)piperidin, in Form der freien Base oder eines kosmetisch verträglichen Salzes.

**27.** Verfahren gemäß Anspruch 22, dadurch gekennzeichnet, daß man als Kuppler eine Verbindung der Formel (I'), worin $R_2$ von einem Wasserstoffatom verschieden ist, oder eines ihrer Salze mit einer Säure zusammen mit mindestens einem wie in Anspruch 26 definierten p-Phenylendiamin verwendet.

**28.** Verfahren gemäß Anspruch 22, dadurch gekennzeichnet, daß man als Kuppler eine Verbindung der Formel (I'), worin $R_2$ ein Wassertoffatom ist, oder eines ihrer Salze mit einer Säure zusammen mit mindestens einem wie in Anspruch 26 definierten p-Phenylendiamin verwendet.

**29.** Verfahren gemäß Anspruch 24, dadurch gekennzeichnet, daß man ein p-Aminophenol verwendet, ausgewählt aus p-Aminophenol, 2-Methyl-4-aminophenol, 3-Methyl-4-aminophenol, 2-Chlor-4-aminophenol, 3-Chlor-4-aminophenol, 2-6-Dimethyl-4-aminophenol, 3, 5-Dimethyl-4-aminophenol, 2, 3-Dimethyl-4-aminophenol, 2, 5-Dimethyl-4-aminophenol, 2-Hydroxymethyl-4-aminophenol, 2-(beta-Hydroxyethyl)-4-aminophenol, 2-Methoxy-4-aminophenol, 3-Methoxy-4-aminophenol.

**30.** Verfahren nach Anspruch 24, dadurch gekennzeichnet, daß man die Oxidationsfarbstoffvorstufe vom para-Typ eine p-substituierte heterozyklische Verbindung verwendet, ausgewählt aus 2,5-Diaminopyridin, 2-Hydroxy-5-aminopyridin und Tetraaminopyrimidin.

**31.** Verfahren gemäß jedem der Ansprüche 22 bis 30, dadurch gekennzeichnet, daß man weitere Kuppler zufügt, ausgewählt aus m-Diphenolen, m-Aminophenolen, m-Phenylendiaminen, m-Acylaminophenolen, m-Ureidophenolen, m-Carbalkoxyaminophenolen, alpha-Naphthol, beta-Ketoverbindungen und Pyrazolonen.

**32.** Verfahren gemäß jedem der Ansprüche 22 bis 31, dadurch gekennzeichnet, daß man ebenfalls Farbstoffvorstufen vom ortho-Typ zufügt, ausgewählt aus o-Aminophenolen, o-Phenylendiaminen und o-Diphenolen.

**33.** Verfahren gemäß jedem der Ansprüche 22 bis 32, dadurch gekennzeichnet, daß man 1 bis 40 Gewichtsprozent eines organischen Lösungsmittels zufügt, ausgewählt aus Niedrigalkanolen, Glyzerin, Glykolen oder Etherglykolen sowie deren Mischungen.

**34.** Verfahren gemäß jedem der Ansprüche 22 bis 33, dadurch gekennzeichnet, daß man 0,5 bis 40 Gewichtsprozent mindestens eines anionischen, kationischen, nicht-ionischen, amphoteren oberflächenaktiven Mittels oder von deren Mischungen zufügt.